# EUROPEAN PATENT APPLICATION

(11) **EP 4 603 108 A1**
(43) Date of publication of application: **20.08.2025**
(21) Application number: 24158478.8
(22) Date of filing: 19.02.2024
(51) Int. Cl.: A61K 47/54, A61K 47/64, A61P 35/00

(54) **HUMAN SERUM ALBUMIN BASED NANOCARRIERS FOR TARGETED IMMUNOTHERAPY**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to a mannose-HSA based nanocarrier system, and a pharmaceutical composition containing the same for delivery of immunomodulatory drugs into target cells expressing surface mannose receptors. The present invention further relates to a method of manufacturing said mannoseHSA nanocarrier system.

## Description

The present invention relates to a mannose-HSA based nanocarrier system, and a pharmaceutical composition containing the same for delivery of immunomodulatory drugs into target cells expressing surface mannose receptors. The present invention further relates to a method of manufacturing said mannose-HSA nanocarrier system.

### Background of the invention

The present disclosure is broadly concerned with drug delivery systems. More particularly, it is concerned with mannose-HSA based protein carrier that carries multiple copies of drugs for immunotherapy.

Immunotherapy has emerged as a new treatment regime for a wide variety of diseases ranging from autoimmune disorders and cancer to fibrosis. In cancer immunotherapies, the major objective is to train the immune system to eliminate cancer cells and to combat metastases. Chronic inflammatory diseases such as liver inflammation can result in fibrosis, cirrhosis and hepatocellular carcinoma (HCC). Vaccination and immune therapy using checkpoint inhibitors or immune system modulators, have been proposed as treatment options. Yet, major drawbacks of small molecule drugs for immunotherapy and chronic inflammatory diseases include low response rate and uncontrolled, systemic immune responses (Rana, I., et al., 2023. Nanocarriers for cancer nano-immunotherapy. Drug Deliv. and Transl. Res. 13, 1936-1954). Consequently, the effects can extend to healthy cells and this leads to adverse side effects throughout a patient's body.

Nano-sized drug delivery systems improve the solubility of drugs, bioavailability and their systemic circulation time. These drug-loaded nanoparticles can reach and accumulate into tumors because of the so-called enhanced permeability and retention (EPR) effect of many solid tumors. Besides tumors, drug-loaded nanoparticles could also accumulate in other types of body tissues that possess an open vasculature, for example in the liver and the spleen, which is a particularly promising target for nanoparticles loaded with immunomodulatory drugs, as they comprise of many antigen presenting cells.

Liposome nanoparticles are the standards in drug delivery, with various systems already approved for use by FDA/EMA, e.g. Doxil,^{®} Comirnaty^{®}. Nevertheless, systemic immune responses and systemic toxicity are still observed in liposome systems. Furthermore, despite preclinical models showing promising results, the clinical efficacy of nano-sized drug delivery is limited due to heterogeneity of the EPR effects, interstitial fluid pressure. Systems to boost uptake of their therapeutic agents into the affected tissues, where it can be most effective, is required. In addition, the controlled release delivery of pharmaceutical agents to targeted sites such as tumors and areas of inflammation is important to prevent unwanted toxicity to healthy cells and tissues.

In immunotherapy, nanoparticle carrier systems are loaded with active ingredients such as mRNA, DNA or an antigen or/and an immunomodulatory drug that can stimulate antigen presentation by target antigen presenting cells to induce a strong and specific activation of anti-tumor T cells. Biopolymers such as proteins and carbohydrates offer biocompatibility and biodegradability and could be good candidates as nanocarriers. Human serum albumin, a major protein in human blood, has been used as a nanocarrier in FDA-approved nanoformulation, Abraxane^{®}. There are various studies which show that albumin is accumulated in tumor-bearing mice. On the other hand, certain immune cells are known to present high density of C-type lectin receptors and this interaction can be exploited to boost the uptake for the active drug ingredient.

Therefore, given the limitations of existing nanocarriers, a targeted drug delivery system and/or composition for immune diseases, that can preferentially deliver high loading of active pharmaceutical ingredients to the diseased sites and release them in a controlled fashion to achieve high efficacy and minimize systemic toxicity, is required. Such a nano-size pharmaceutical composite should direct the accumulation of a drug directly into the affected tissue in a highly concentrated manner for immunotherapy.

The present invention solves the above problem by providing a fully biopolymer-based nanocarrier with excellent performance that addresses previous limitations and enables targeted modulation of a set of immune cells, which play a key role in many immune-related conditions.

The objective of the present invention is solved by the teaching of the independent claims. Further advantageous features, aspects and details of the invention are evident from the dependent claims, the description, the figures, and the examples of the present application.

### Brief description of the invention

The present invention is directed to an HSA nanocarrier system for drug delivery into a target cell, wherein said nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering.

In preferred embodiments, the at least one immunomodulatory drug is covalently bound the HSA, when the HSA is in a denatured state and afterwards backfolded by rapid dilution. Thus, the present invention is also directed to an HSA nanocarrier system for drug delivery into a target cell, comprising:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the HSA bound to the plurality of mannose-based targeting structures and to the at least one immunomodulatory drug is backfolded by a rapid dilution step.

In another embodiment, the HSA nanocarrier system comprises a mannose-based targeting structure selected from the group consisting of Man*p*N₃, ManpsNs, (Man*p*)*₃*N₃, and (Man*p₃*)₃N₃. Thus, the present invention is also directed to an HSA nanocarrier system for drug delivery into a target cell, comprising:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the mannose-based targeting structure of the plurality of mannose-based targeting structures is selected from the group consisting of Man*p*N₃, ManpsNs, (Man*p*)*₃*N₃, and (Man*p₃*)₃N₃.

Most preferably, the mannose-based targeting structure is Man*p₃*N₃ (trimannose).

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the mannose-based targeting structure of the plurality of mannose-based targeting structures is selected from the group consisting of Man*p*N₃, ManpsNs, (Man*p*)*₃*N₃, and (Man*p₃*)₃N₃ and wherein the HSA bound to the plurality of mannose-based targeting structures and to the at least one immunomodulatory drug is backfolded by a rapid dilution step.

In another embodiment, the HSA nanocarrier system comprises a molar immunomodulatory drug:HSA ratio between 1 and 30. Thus, the present invention is also directed to an HSA nanocarrier system for drug delivery into a target cell, comprising:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the molar immunomodulatory drug:HSA ratio is comprised between 1 and 30

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the molar immunomodulatory drug:HSA ratio is comprised between 1 and 30, and wherein the mannose-based targeting structure of the plurality of mannose-based targeting structures is selected from the group consisting of Man*p*N₃, Man*p₃*N₃, (Man*p*)*₃*N₃.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the molar immunomodulatory drug:HSA ratio is comprised between 1 and 30, and wherein the mannose-based targeting structure of the plurality of mannose-based targeting structures is selected from the group consisting of Man*p*N₃, ManpsNs, (Man*p*)*₃*N₃, and (Man*p₃*)₃N₃ and wherein the HSA bound to the plurality of mannose-based targeting structures and to the at least one immunomodulatory drug is backfolded by a rapid dilution step.

In another embodiment, the HSA nanocarrier system comprises a plurality of mannose-based targeting structures, wherein the molar mannose-based targeting structure:HSA ratio is comprised between 1 and 59. Thus, the present invention is also directed to an HSA nanocarrier system for drug delivery into a target cell, comprising:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the molar mannose-based targeting structure:HSA ratio is comprised between 1 and 59.

In another embodiment, the HSA nanocarrier system comprises a plurality of mannose-based targeting structures covalently bound to the HSA via an interconnecting molecule, wherein the molar ratio of interconnecting molecule:HSA is comprised between 3 and 59, and/or wherein the molar mannose-based targeting structure:HSA ratio is comprised between 1 and 59. Thus, the present invention is also directed to an HSA nanocarrier system for drug delivery into a target cell, comprising:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59

In a preferred embodiment, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the plurality of mannose-based targeting structures is covalently bound to the HSA via an interconnecting molecule and the molar ratio of interconnecting molecule:HSA is comprised between 3 and 59, and wherein the molar mannose-based targeting structure:HSA ratio is comprised between 1 and 59

In a preferred embodiment, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the plurality of mannose-based targeting structures is covalently bound to the HSA via an interconnecting molecule, and wherein the molar mannose-based targeting structure:HSA ratio is comprised between 1 and 59

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the plurality of mannose-based targeting structures is covalently bound to the HSA via an interconnecting molecule and the molar ratio of interconnecting molecule:HSA is comprised between 3 and 59, wherein the molar mannose-based targeting structure:HSA ratio is comprised between 1 and 59, wherein the molar immunomodulatory drug:HSA ratio is comprised between 1 and 30, and wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the plurality of mannose-based targeting structures is covalently bound to the HSA via an interconnecting molecule and the molar ratio of interconnecting molecule:HSA is comprised between 3 and 59, wherein the molar mannose-based targeting structure:HSA ratio is comprised between 1 and 59, wherein the molar immunomodulatory drug:HSA ratio is comprised between 1 and 30, and wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

In another embodiment, the HSA nanocarrier system comprises at least one immunomodulatory drug selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin. Thus, the present invention is also directed to an HSA nanocarrier system for drug delivery into a target cell, comprising:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, and wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, and wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

In another embodiment, the HSA nanocarrier system comprises at least one immunomodulatory drug covalently bound to said HSA by a drug linker using a thiol-reactive functional group selected from the group consisting of maleimide, iodoacetamide, and iodoacetate. Thus, the present invention is also directed to an HSA nanocarrier system for drug delivery into a target cell, comprising:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the immunomodulatory drug is covalently bound to said HSA by a drug linker using a thiol-reactive functional group selected from the group consisting of maleimide, iodoacetamide, and iodoacetate.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a drug linker using a thiol-reactive functional group selected from the group consisting of maleimide, iodoacetamide, and iodoacetate, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a drug linker using a thiol-reactive functional group selected from the group consisting of maleimide, iodoacetamide, and iodoacetate, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a drug linker using a thiol-reactive functional group selected from the group consisting of maleimide, iodoacetamide, and iodoacetate, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a drug linker using a thiol-reactive functional group selected from the group consisting of maleimide, iodoacetamide, and iodoacetate, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a drug linker using a thiol-reactive functional group selected from the group consisting of maleimide, iodoacetamide, and iodoacetate, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

In another embodiment, the HSA nanocarrier system comprises at least one immunomodulatory drug covalently bound to said HSA by a traceless, self-immolative drug linker comprising a thiol-reactive functional group, wherein the traceless, self-immolative linker is selected pyridinyldithiol-ethoxy-carbonyl or mc-vc-pabc linker (maleimidocaproyl (mc) - valine-citrulline (vc) -para-amino benzyloxycarbonyl (PABC)). Thus, the present invention is also directed to an HSA nanocarrier system for drug delivery into a target cell, comprising:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the immunomodulatory drug is covalently bound to said HSA by a traceless, self-immolative drug linker comprising a thiol-reactive functional group, wherein the traceless, self-immolative linker is selected pyridinyldithiol-ethoxy-carbonyl or mc-vc-pabc linker (maleimidocaproyl (mc) - valine-citrulline (vc) -para-amino benzyloxycarbonyl (PABC)).

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a traceless, self-immolative drug linker comprising a thiol-reactive functional group, wherein the traceless, self-immolative linker is covalently bound to a ketone or aldehyde group of the immunomodulatory drug and the linker is selected from methanethiosulfonate, thiosulfonate, and maleimide-hydrazide (e.g. BMPH (N-β-maleimidopropionic acid hydrazide)), wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a traceless, self-immolative drug linker comprising a thiol-reactive functional group, wherein the traceless, self-immolative linker is covalently bound to a ketone or aldehyde group of the immunomodulatory drug and the linker is selected from methanethiosulfonate, thiosulfonate, and maleimide-hydrazide (e.g. BMPH (N-β-maleimidopropionic acid hydrazide)), and wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a traceless, self-immolative drug linker comprising a thiol-reactive functional group, wherein the traceless, self-immolative linker is covalently bound to a ketone or aldehyde group of the immunomodulatory drug and the linker is selected from methanethiosulfonate, thiosulfonate, and maleimide-hydrazide (e.g. BMPH (N-β-maleimidopropionic acid hydrazide)), wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a traceless, self-immolative drug linker comprising a thiol-reactive functional group, wherein the traceless, self-immolative linker is covalently bound to a ketone or aldehyde group of the immunomodulatory drug and the linker is selected from methanethiosulfonate, thiosulfonate, and maleimide-hydrazide (e.g. BMPH (N-β-maleimidopropionic acid hydrazide)), wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

Preferably, the HSA nanocarrier system for drug delivery into a target cell, comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein HSA is linked to the mannose-based targeting structure by an azide-reactive interconnecting molecule selected from the group consisting of DBCO, Endo-BCN, DIBO, DIFO, BCN.

In a preferred embodiment, the HSA nanocarrier system further comprises a fluorescent dye. Thus, the HSA nanocarrier system according to the invention for drug delivery into a target cell, comprises preferably:
A) a human serum albumin (HSA);
B) a plurality of mannose-based targeting structures covalently bound to said HSA;
C) at least one immunomodulatory drug covalently bound to said HSA;
D) a fluorescent dye covalently bound to said HSA, wherein the fluorescent dye comprises a maleimide as thiol-reactive functional group or an NHS-ester as amine-reactive functional group, and wherein the fluorescent dye emits light in a range of 400 nm to 850 nm.
wherein said HSA nanocarrier system has a diameter in the range comprised between 4 nm and 150 nm, as measured by dynamic light scattering.

Preferably, the HSA nanocarrier system according to the invention for drug delivery into a target cell, comprises:
A) a human serum albumin (HSA);
B) a plurality of mannose-based targeting structures covalently bound to said HSA;
C) at least one immunomodulatory drug covalently bound to said HSA;
D) a fluorescent dye covalently bound to said HSA, wherein the fluorescent dye comprises a maleimide as thiol-reactive functional group, and wherein the fluorescent dye emits light in a range of 400 nm to 850 nm.
wherein said HSA nanocarrier system has a diameter in the range comprised between 4 nm and 150 nm, as measured by dynamic light scattering, and wherein the fluorescent dye is selected from the group consisting of sulfo-cyanin-maleimide, Alexa Fluor-maleimide, and cyanin-maleimide.

The inventive HSA nanocarrier system is particularly effective in targeting cells expressing a mannose surface receptor. Therefore, the present invention is also directed to an HSA nanocarrier system for drug delivery into a target cell expressing a mannose surface receptor, comprising:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering.

In a preferred embodiment, the present invention is directed to an HSA nanocarrier system for drug delivery into a target cell expressing, comprising:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the target cells express a mannose surface receptor selected from the group consisting of CD206, CD209, and homologues thereof.

In a further preferred embodiment, the present invention is directed to an HSA nanocarrier system for drug delivery into a target cell expressing, comprising:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the target cells are selected from the group consisting of dendritic cells, macrophages, B cells, T cells, liver non-parenchymal cells, splenic cells, neutrophils, and cultured cells expressing an exogenous surface mannose receptor.

A particularly preferred embodiment is directed to an HSA nanocarrier system as described herein, wherein said HSA nanocarrier system comprises:
- trimannose bound via a NHS-PEGn-DBCO interconnecting molecule to HSA at a molar trimannose:HSA range comprised between 1 and 59;
- Cucurbitacin B or TLR7/8 agonist as an immunomodulatory drug at a molar drug:HSA range preferably comprised between 5 and 27, wherein the immunomodulatory drug is covalently bound to said HSA by a traceless, self-immolative pyridinyldithio-ethoxy-carbonyl drug linker;
- optionally a sulfo-Cyanin5-maleimide dye or a sulfo-Cyanin7.5-maleimide dye conjugated to said HSA
and wherein said HSA nanocarrier system has a diameter in the range comprised between 4 nm and 150 nm.

Another aspect of the present invention is directed to a pharmaceutical composition comprising the HSA nanocarrier system as described herein together with a pharmaceutical acceptable excipient.

Another aspect of the present invention is directed to a method to manufacture a HSA nanocarrier system as described herein, wherein the method comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of an immunomodulatory drug to said denatured mannosylated HSA, wherein the immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system.

In preferred embodiments of the method to manufacture a HSA nanocarrier system as described herein, the monomolecular HSA nanocarrier system has a molar mannose:HSA ratio comprised between 1 and 59, and/or a molar interconnecting molecule:HSA ratio comprised between 3 and 59.

In preferred embodiments of the method to manufacture a HSA nanocarrier system as described herein, the mannose-based targeting structure is selected from the group consisting of Man*p*N₃, ManpsNs, (Man*p*)*₃*N₃, and (Man*p₃*)₃N₃.

In preferred embodiments of the method to manufacture a HSA nanocarrier system as described herein, the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

In preferred embodiments of the method to manufacture a HSA nanocarrier system as described herein, the molar immunomodulatory drug:HSA ratio is comprised between 1 and 30.

In preferred embodiments of the method to manufacture a HSA nanocarrier system as described herein, the immunomodulatory drug is covalently bound to said HSA by a traceless, self-immolative linker comprising a thiol-reactive functional group. Preferably, the traceless, self-immolative linker is selected from pyridinyldithio-ethoxy-carbonyl, or mc-vc-pabc linker (maleimidocaproyl (mc) - valine-citrulline (vc) -para-amino benzyloxycarbonyl (PABC).

In preferred embodiments of the method to manufacture a HSA nanocarrier system as described herein, the drug linker is selected from the group consisting of pyridinyldithio-ethoxy-carbonyl-group, maleimide, mc-vc-pabc linker, thiosulfonate linker, hydrazide-maleimide linker, iodoacetamide linker, and methanethiosulfonate linker.

In preferred embodiments of the method to manufacture a HSA nanocarrier system as described herein, the interconnecting molecule in step ii. comprises an azide-reactive group selected from DBCO, Endo-BCN, DIBO, DIFO, and BCN.

In preferred embodiments of the method to manufacture a HSA nanocarrier system as described herein, the HSA is labeled with a fluorescent dye emitting fluorescence in a range comprised between 400 and 850 nm.

In preferred embodiments of the method to manufacture a HSA nanocarrier system as described herein, the HSA is labeled with a fluorescent dye via a maleimide or an NHS-ester.

In a preferred embodiment, the method to manufacture a HSA nanocarrier system as described herein, comprises the following steps:
i.) providing monomolecular native HSA, wherein the monomolecular native HSA is optionally labeled with a fluorescent dye;
i.1) subjecting the HSA labeled with a fluorescent dye maleimide to a ring-opening hydrolysis by adding a buffer at a pH above 9.
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of an immunomodulatory drug to said denatured mannosylated HSA, wherein the immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system.

### Detailed description of the Invention

The present invention provides a novel nanocarrier system based on human serum albumin (HSA) to target certain immune cells for immunotherapy to treat cancer, and other diseases such as liver fibrosis.

The novel nanocarrier system comprises HSA, a mannose-based targeting structure, such as a trimannose (TM), and at least one immunomodulatory drug covalently bound to said HSA. The at least one immunomodulatory drug is preferably linked to HSA via a traceless linker.

The inventive nanocarrier system has the advantage that the drug cargo is specifically and efficiently delivered via active targeting to target cells expressing surface mannose receptors, such as CD209 or CD206 or homologues thereof in the case of a trimannose.

Moreover, the inventive nanocarrier system has the advantage over the prior art that the drug-cargo is covalently linked to HSA.

In an exemplary synthesis procedure, the lysine residues of native HSA are modified using NHS-chemistry to attach DBCO (Dibenzoazacyclooctyne) linkers to the protein surface. The glycolytic targeting structure, e.g. trimannose (TM), is synthesized with an azide as functional group to click in a strain-promoted copper free azide alkyne click reaction to the DBCO linkers. The mannosylated HSA is then completely denatured including the reduction of the native disulfides. The resulting free thiols react with a thiol-reactive functional group attached to at least one immunomodulatory drug of interest. Preferred thiol-reactive functional groups to attach the immunomodulatory drug are selected from a pyridinyldithio-ethoxy-carbonyl-group or a maleimide. Unreacted thiols on HSA are capped with a capping reagent, such as *N*-ethylmaleimide. Afterwards, all reactive substances are washed out and the conjugated HSA protein is folded via rapid dilution procedure, thereby leading to an α-helix-rich globular structure very similar to the native conformation based on CD measurements. The synthesis procedures allow obtaining nanocarrier preparations containing monomolecular HSA-nanocarriers and only less than 8% aggregates or multi-HSA nanocarriers based on FCS measurements.

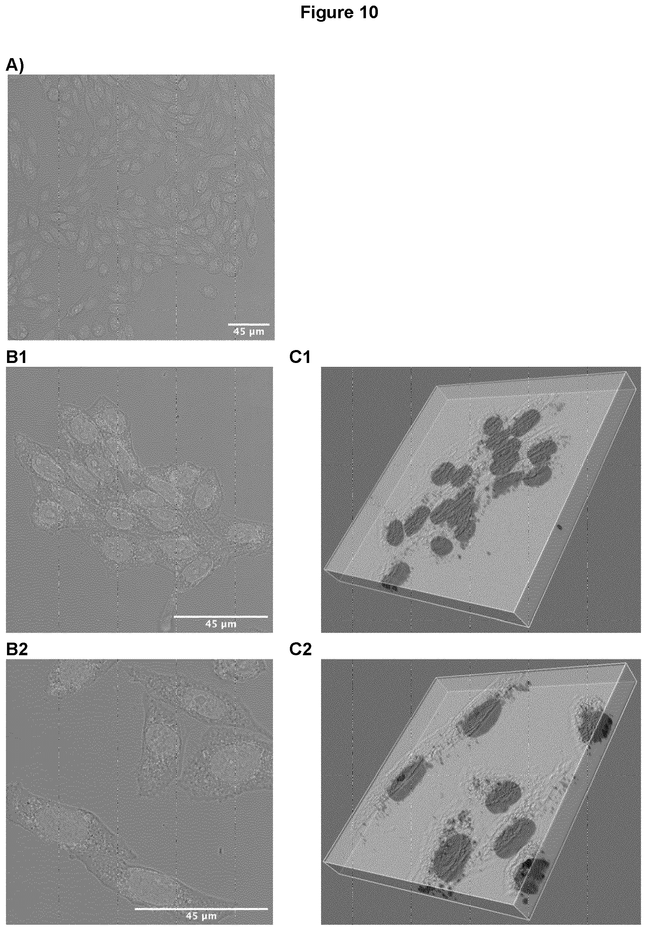

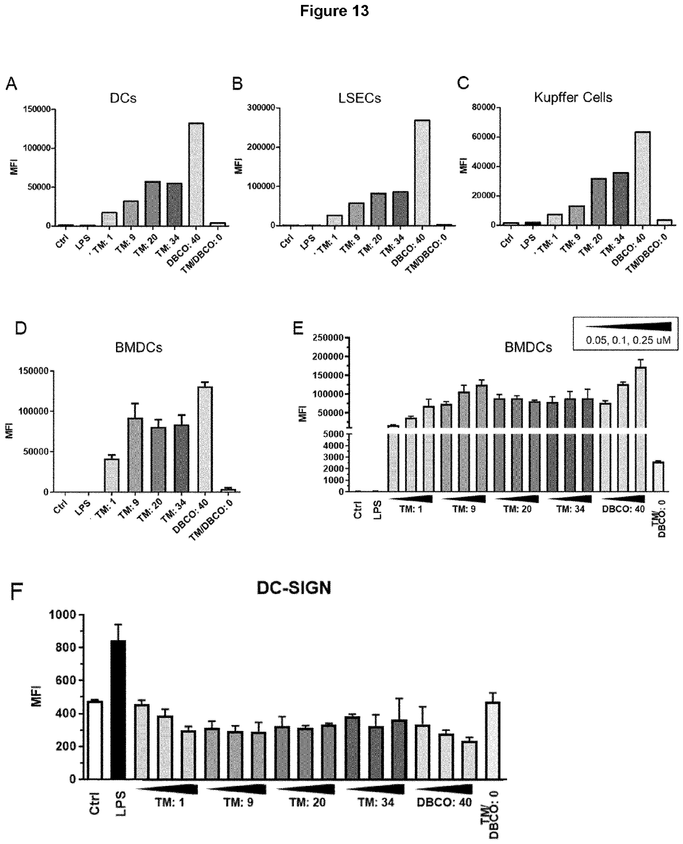

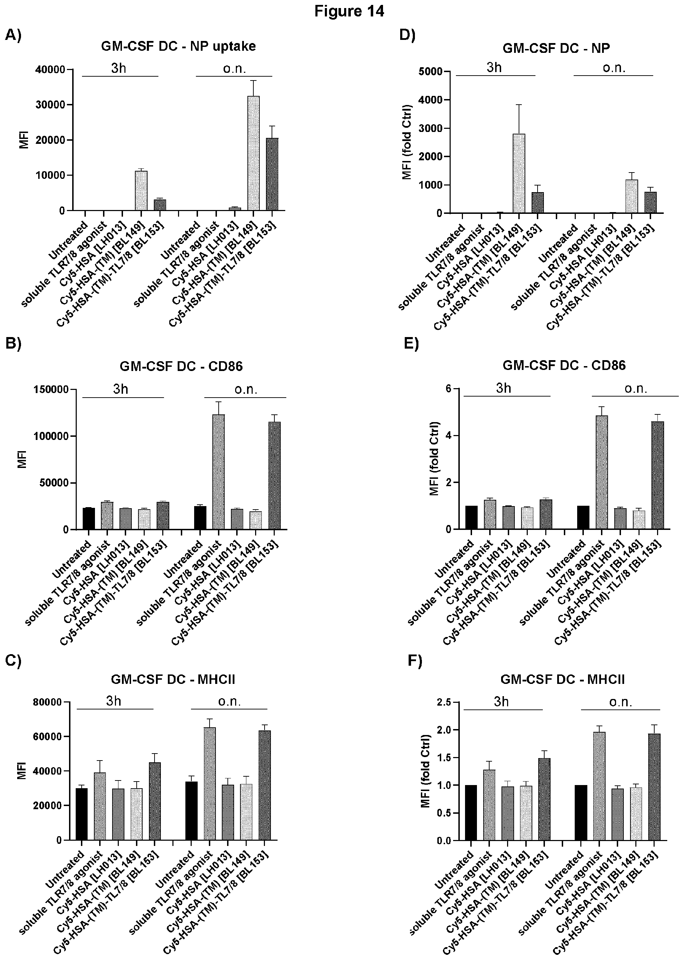

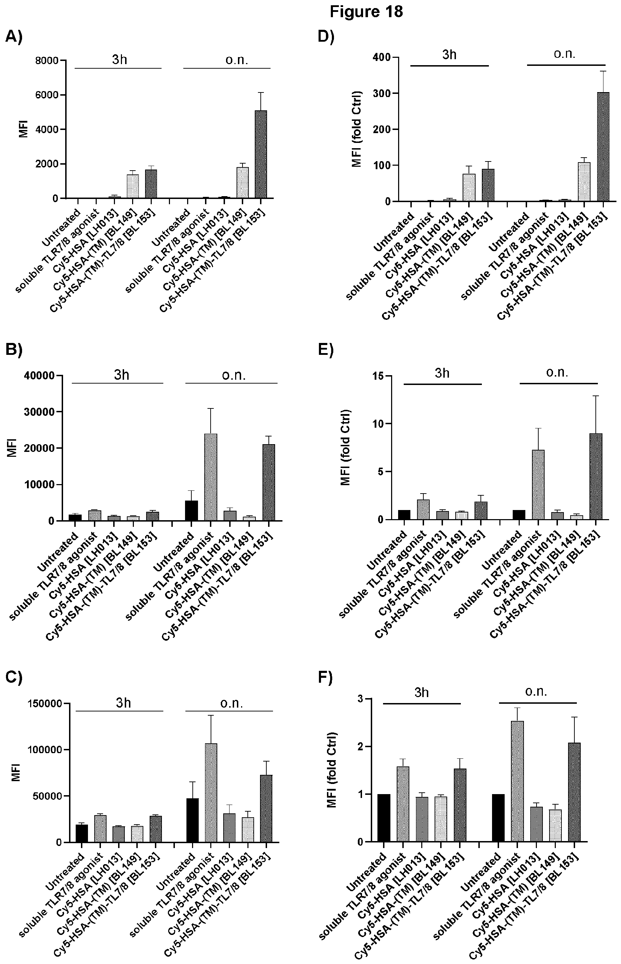

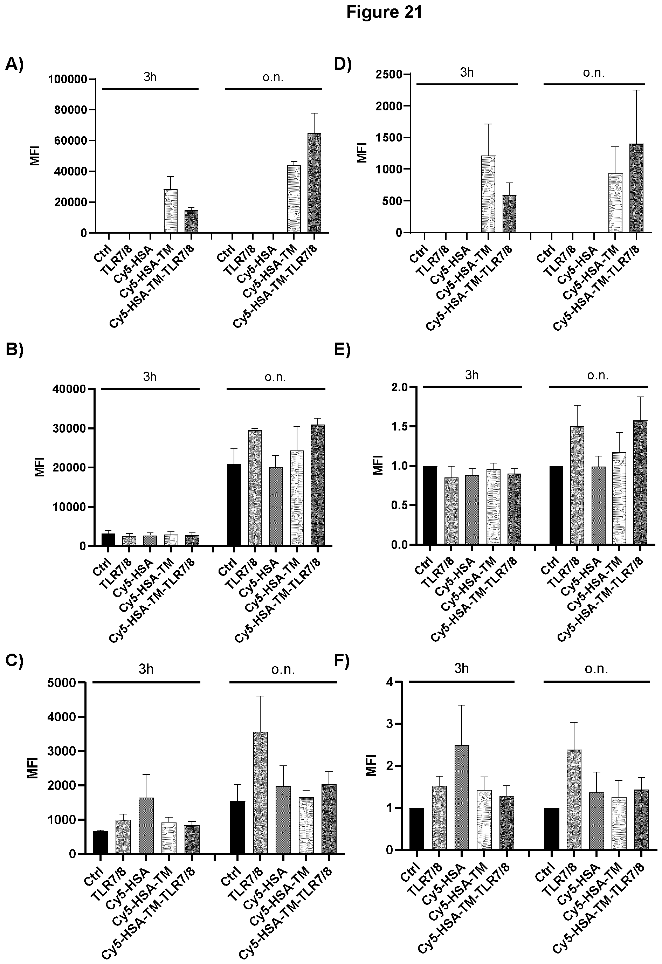

*In vitro* assays showed excellent uptake of this novel nanocarrier system compared to suitable controls by CD206-transfected CHO-cells (Figures 10-12), GM-CSF differentiated BMDC (Figures 13-16, and 30), FLT3L differentiated BMDC (Figures 18-20), liver NPC cells (Figures 13, and 26-28), and spleen cells (Figures 21-25) in 2D-*in vitro* cell uptake studies. These experiments also showed no immune stimulatory effect by the carrier itself or its substructures without the drug cargo. Moreover, performed experiments showed immune stimulation of antigen presenting cells (Figures 14- 19, 21 - 23, and 25).

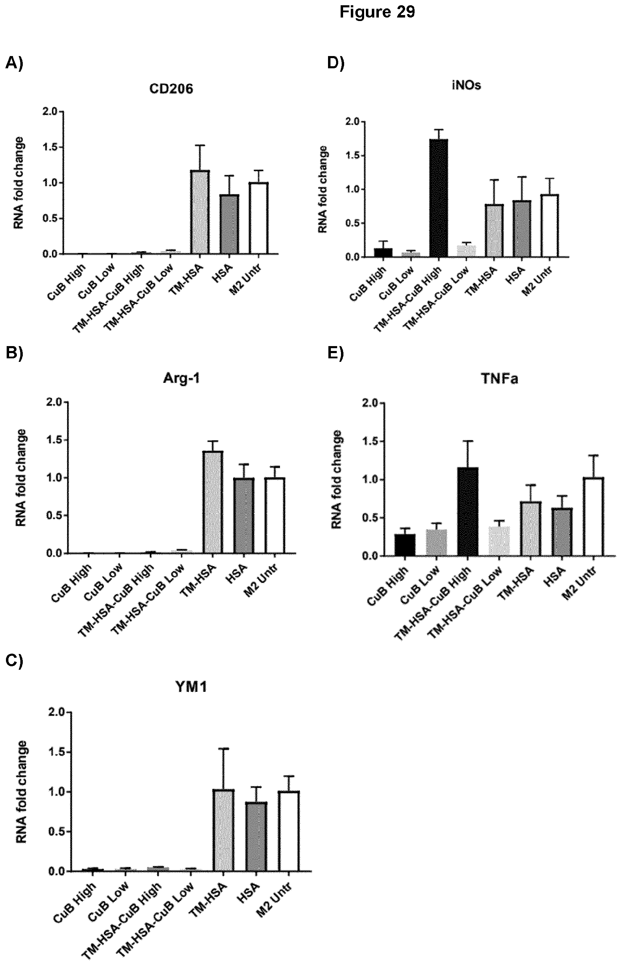

Moreover, TM-HSA nanocarriers loaded with Cucurbitacin B were able to induce phenotype-repolarization of M2-type BMDM (Figure 29).

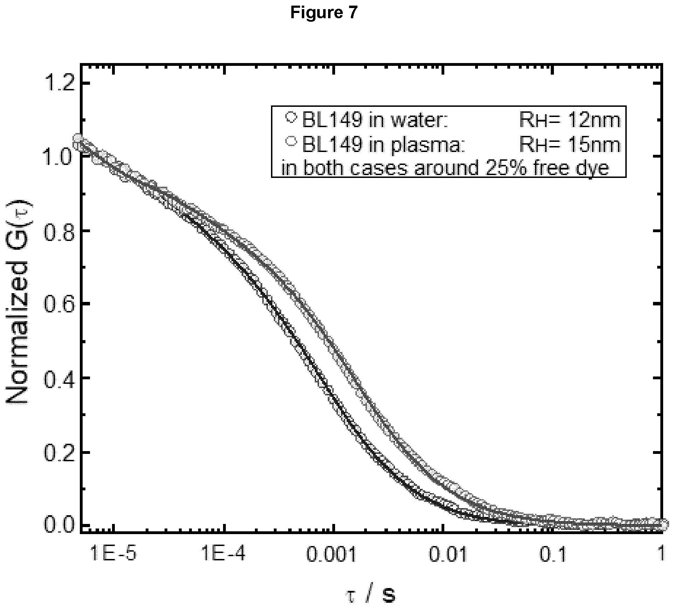

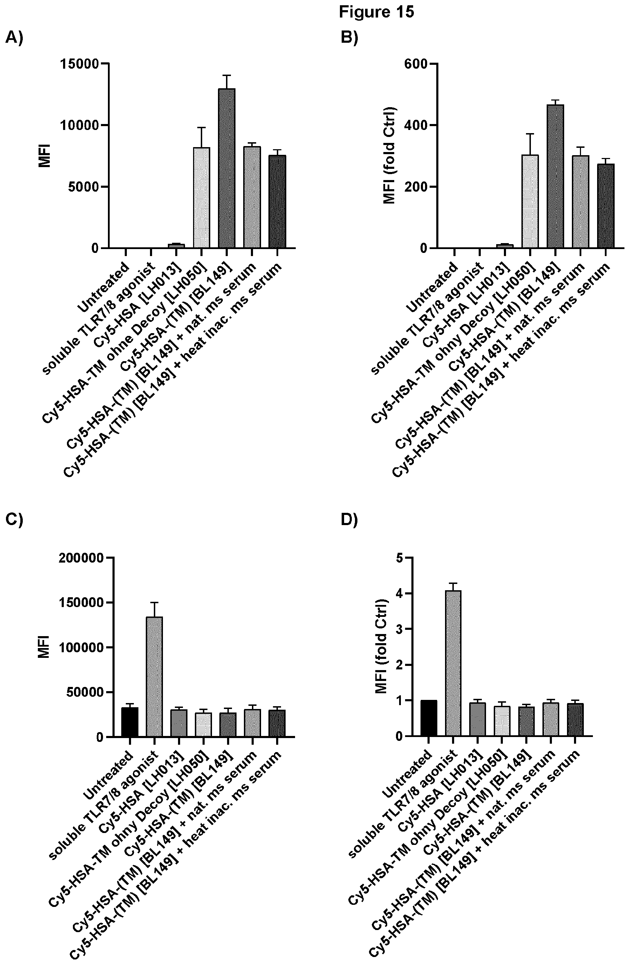

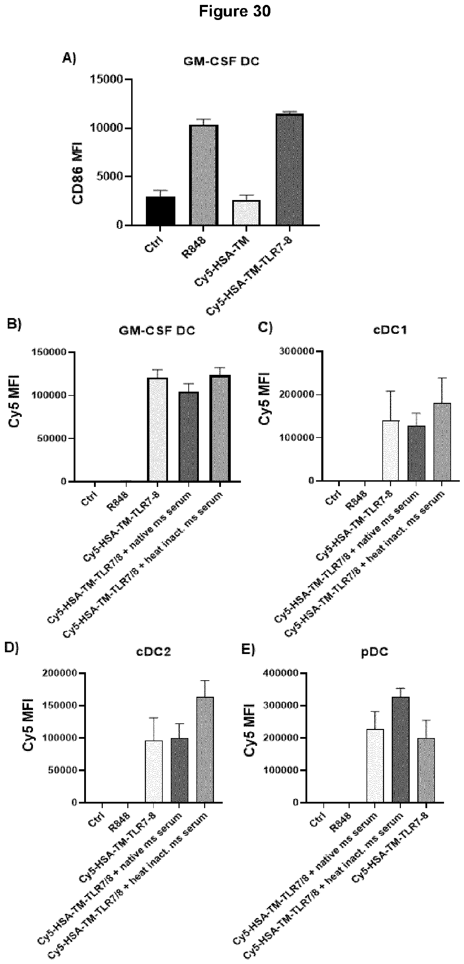

Uptake experiments with HSA-nanocarriers preincubated with native and heat-inactivated mouse serum demonstrated that uptake is not significantly decreased indicating that no major protein corona is formed in presence of serum, which could hamper drug delivery (Figures 15, 30). This is further supported by FCS experiments showing no major increase in hydrodynamic radius of benzyl (decoy) loaded HSA nanocarrier upon preincubation with blood plasma (Figure 7).

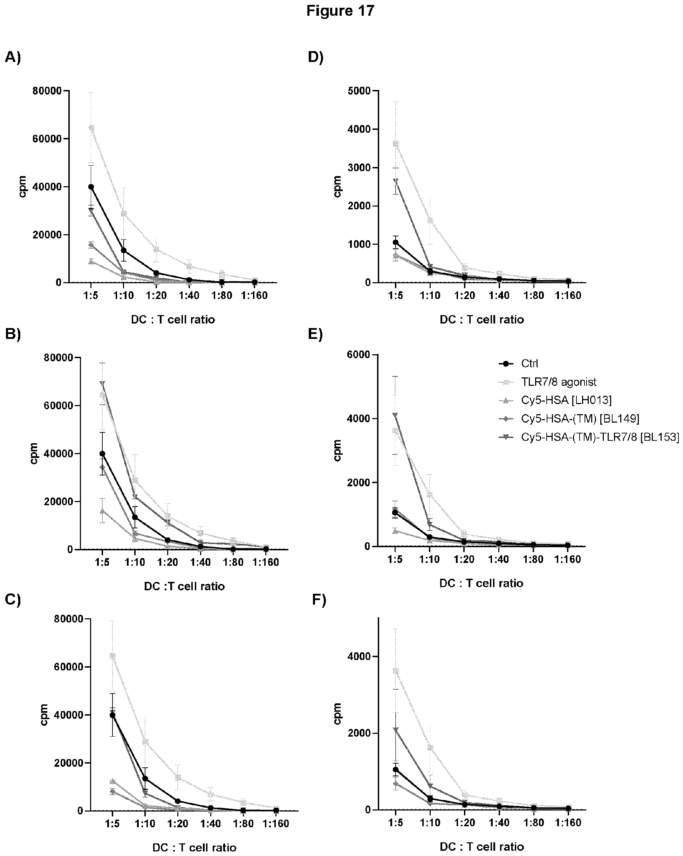

TLR7/8-Agonist loaded HSA nanocarrier, in contrast to unloaded or decoy loaded HSA nanocarriers, showed equal to superior immune stimulation compared to equimolar small molecule treatment which is demonstrated by FACS analysis of surface activation markers (CD86, MHCII) of respective immune cells upon nanocarrier treatment (Figure 14, 15, 18-28) and by T-cell proliferation assay (Figure 17).

Human Serum Albumin (HSA), the biopolymeric carrier material, is biocompatible, biodegradable, has low immunogenicity and is FDA approved in nanoparticle formulations like Abraxane^{®} (ca. 140 nm). HSA is a well-known transportation protein, is ubiquitous in blood plasma, comprises natural, non-toxic monomers; and has low membrane permeability.

Native HSA has 35 cysteine (-SH) residues (including disulfide bridges), 59 lysine (-NH₂) residues, and 100 aspartic/glutamic acid (-COOH) residues.

The term "monomolecular mannosylated HSA nanocarrier system" as used herein refers to a nanoparticle comprising one HSA, a plurality of mannose-based targeting structures, and an immunomodulatory drug of choice, all covalently linked or conjugated. The HSA in the nanocarrier has preferably an α-helix rich, globular conformation.

The term "HSA nanocarrier system" or "nanocarrier system" as used herein refers to a nanoparticle comprising one HSA, a single or a plurality of mannose-based targeting structures, and at least one immunomodulatory drug, wherein the mannose-based targeting structures and the at least one immunomodulatory drug are covalently linked or conjugated to HSA. The mannose-based targeting structures and the at least one immunomodulatory drug can either be directly bound to the HSA or via a linker/spacer. However, the mannose-based targeting structures are not coupled to the at least one immunomodulatory drug and the at least one immunomodulatory drug is not coupled to the mannose-based targeting structures. Therefore, the mannose-based targeting structures and the at least one immunomodulatory drug are covalently linked or conjugated to HSA such that there is no interaction between the mannose-based targeting structures and the at least one immunomodulatory drug. The HSA in the nanocarrier system has preferably an α-helix rich, globular conformation.

The term "monomolecular HSA nanocarrier system" as used herein refers to a HSA nanocarrier system as described herein comprising a single HSA molecule in its monomeric form, without covalent dimers, trimers, or other higher order oligomers.

The term "ring-opening hydrolysis" refers to a hydrolysis reaction to open a maleimide ring occurring at high pH (above 9). The ring-opening reaction is irreversible and leads to stable products.

### Fluorescent dyes to trace the HSA nanocarrier system

According to one aspect of the invention, the HSA nanocarrier system is traced by conjugating the HSA molecule with a fluorescent dye. Preferred dyes are water soluble dyes emitting in the range 400 - 850 nm (VIS/NIR range). Preferred dyes are sulfocyanine dyes, or other derivatives of dyes of the coumarin, rhodamine, cyanine or xanthene family.

**Sulfo-Cyanine5-Maleimide** (Mal-SCy5) is a water soluble, hydrophilic dye similar to Cy5^{®} maleimide (Cy5^{®} maleimide analog) for protein labeling, including labeling of antibodies. Labeled proteins can be easily separated from unreacted dye by gel filtration, spin column purification, dialysis, electrophoresis or chromatography. **Cyanine5-Maleimide** is a mono-reactive dye which selectively couples with thiol groups (for example, with cysteines in peptides and proteins) to give labeled conjugates. Cyanine5 is an analog of Cy5^{®} (2-[5-[1-(5-Carboxypentyl)-1,3-dihydro-3,3-dimethyl-5-sulfo-2H-indol-2-ylidene]-1,3-pentadienyl]-1-ethyl-3,3-dimethyl-5-sulfo-3H-indolium inner salt), a common fluorophore which is compatible with various instrumentations like microscopes, imagers, and fluorescence readers.

### Mannose-based targeting structures

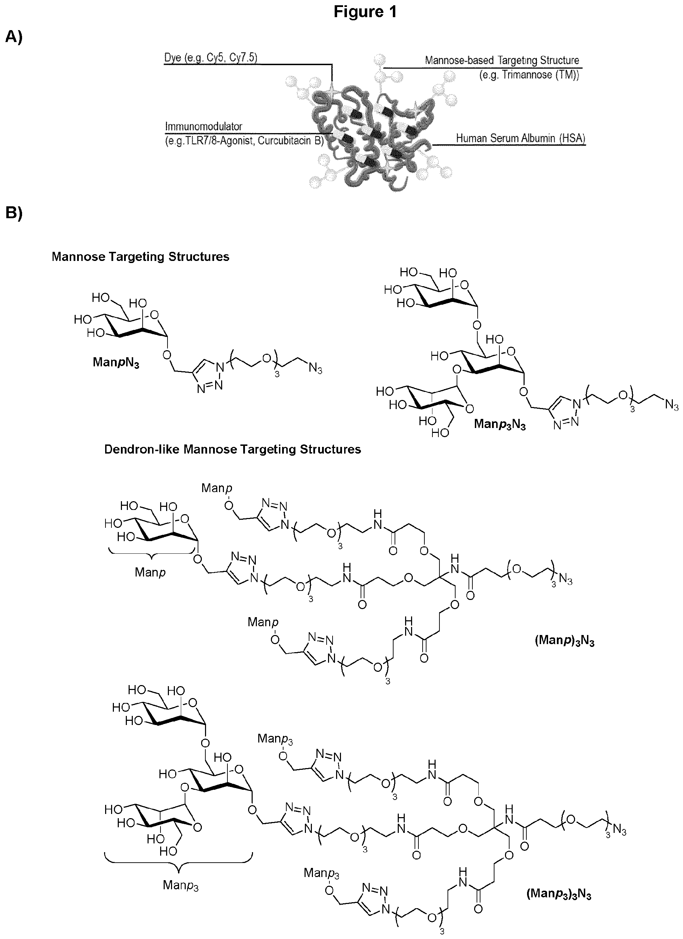

The term **"mannose-based targeting structure"** as used herein refers to an antennary molecular structure comprising one or more terminal mannose saccharides, wherein the reducing end of each mannose saccharide is functionalized with one and the same backbone forming linker comprising a functional group, such as a terminal azide group, for covalent attachment to the nanocarrier for instance via an azide-alkyne-click-reaction. In case of more than one terminal mannose saccharide, the linker is a branched molecule connecting each terminal mannose saccharide with the functional group as focal point, thereby forming a dendron-like structure. Each terminal mannose saccharide consists of one or more mannopyranose units, wherein, when the terminal mannose saccharide comprises more than one mannopyranose unit, these mannopyranose units are bound to each other via a glycosidic bond (e.g. an α-(1→3)- and/or an α-(1→6)-glycosidic bond). Exemplarily, the monosaccharide targeting structure Man*p*N₃ consists of a single α-*O*-mannopyranoside unit and a tetraethylene glycol spacer, which contains an azide function at the terminal position. Both parts are connected via a 1 ,4-disubstituted-1 ,2,3-triazole, with position 1 connected to the spacer and position 4 to a glycosylated oxo-methylene group. The trisaccharide targeting structure Man*p*₃N₃ (TM) comprises a central α-*O*-mannopyranoside unit carrying the same spacer aglycon as described for Man*p*N₃, and two further mannopyranosyl residues attached to the central unit via an α-(1→3)- and an α-(1→6)-glycosidic bond, respectively. The monosaccharide carrying dendron-like targeting structure (Man*p*)₃N₃ contains three α-*O*-mannopyranoside units each glycosylated to a triazole tetraethylene glycol spacer, as the ones already described. The spacers themselves are connected via a terminal amino group as part of an amide bond to a central structure, containing three ethylene units branching out from the TRIS-based core. The amino group of the core is connected via an amide bond to a triethylene glycol-based linker, whose acid function results from a Michael addition with tert-butyl acrylate, and which carries the terminal azide group for attachment to the nanocarrier. The trisaccharide carrying dendron-like targeting structure (Man*p*₃)₃N₃ contains three trimannose units, as described for ManpsNs, each glycosylated in the same way as described for (Man*p*)₃N₃. A preferred "mannose-based targeting structure" for the HSA nanocarrier system disclosed herein is selected from the group consisting of the described targeting structures Man*p*N₃, Man*p*₃N₃, (Man*p*)*₃*N₃, and (Man*p₃*)₃N₃ (Figure 1B).

The biological targeting unit mannose, and in particular trimannose (TM), binds to C-type lectin receptors, namely CD206 (MMR) and CD209 (DC-SIGN) and respective homologues (Zhang F, et al., 2014. DC-SIGN, DC-SIGNR and LSECtin: C-type lectins for infection. Int Rev Immunol. 33(1):54-66; Boskovich et al., 2006.

Protein Structure and Folding 281(13) P8780-8787; Gupta et al., 2012. Animal Lectins: Form, Function and Clinical Applications, 773-798.). MMR is mainly expressed on M2-polarized macrophages, which are anti-inflammatory and with pro-tumorous phenotype, immature dendritic cells as well as specialized liver endothelial cells LSECs. DC-SIGN is mainly expressed on dendritic cells.

The term **"molar ratio mannose:HSA"** or synonymously "molar mannose:HSA ratio" refers to the number of molecules of mannose loaded on one HSA molecule.

### Linkage of mannose to HSA

The mannose-based targeting structure is linked to HSA by using click chemistry, preferably by copper free azide-alkyne click chemistry, thereby forming mannosylated HSA.

Suitable linkers reacting with azide are preferably selected from NHS-PEGₙ-DBCO, Endo-BCN-PEGₙ-NHS, NHS-PEGₙ-DIBO, NHS-PEGₙ-DIFO, NHS-PEG_{N}-BCN, NHS-DBCO, Amine-PEGₙ-DBCO, Amine-DBCO,Acid-PEGₙ-DBCO, Acid-DBCO, STP-PEGₙ-DBCO, STP-DBCO, TFP-DBCO, TFP-PEGₙ-DBCO, PTP-PEG_{N}-DBCO, PTP-DBCO, wherein
DBCO (or DIBAC or ADIBO) stands for dibenzoazacyclooctyne, Endo-BCN for endo-bicyclo[6.1.0]non-4-yn-9-yl, DIBO for dibenzocyclooctyne, DIFO for difluorocyclooctyne-CH₂-COOH, and BCN for bicyclo[6.1.0]nonyne.

NHS-PEGn-DBCO refers to a dibenzoazacyclooctyne polyethylene glycol NHS-ester, wherein n represents the number of ethylene glycol repeating units. Preferably, n is an integer selected between 1 and 50, more preferably between 1 and 20, more preferably between 2 and 10, more preferably between 2 and 8. In another embodiment, n is 3. In a preferred embodiment, n is 4. In another embodiment, n is 6. In another embodiment, n is 12. In another embodiment, n is 24.

The term **"mannosylated HSA"** as used herein refers to a conjugate of one or more mannose-based targeting structures and HSA. The HSA may be labeled with a fluorescent dye.

Unreacted thiols on HSA at step v. can be capped with any maleimide, preferably small, n-substituted maleimides. Preferably, unreacted thiols on HSA are capped with N-Ethylmaleimide

The term **"linker"** as used herein encompasses molecular fragments capable of connecting the reducing-end monosaccharide of the mannose-based targeting structure with a reactive group of HSA, optionally by binding to at least one interconnecting molecule. Thus, the function of the linker *per se* or together with the interconnecting molecule is to establish, keep and/or bridge a special distance between the reducing-end mannose and HSA. More specifically, one extremity of the linker is connected to the exocyclic oxygen atom at the anomeric center of the reducing-end mannose and the other extremity is connected *via* the functional group, e.g. an azide with the interconnecting molecule, or directly with HSA.

As used herein, the term **"interconnecting molecule"** refers to a bifunctional molecule containing functional group X and functional group Y, wherein functional group X is capable of reacting with the terminal functional group on the linker and the functional group Y is capable of reacting with a functionality present on HSA, preferably a primary amino group, such as an ε-amino group of lysine. A preferred interconnecting molecule used in the inventive nanocarrier system is NHS-PEGn-DBCO, particularly, NHS-PEG₄-DBCO.

### Immunomodulatory drug cargo

The term "immunomodulatory drug" or "immunomodulatory compound" or "immunomodulator" as used herein refers to a small molecule or peptidic inhibitor that affects the functioning of the immune system by increasing or decreasing the immune response.

Preferred immunomodulatory drugs for the nanocarrier system disclosed herein are small molecules or peptidic inhibitors selected from the group consisting of TLR agonists (e.g.TLR7/8-agonist R848 derivative or RGPPP peptide from *Pseudostellaria heterophylla*)*,* STING agonists (e.g. CL845, STG-982, STG-968 (Invivogen)), PARP inhibitors, JAK inhibitor (e.g. CuB), STAT3 inhibitors (e.g. CuB), PI3K Inhibitor (e.g. CuB) ERAP2 inhibitor (e.g. navoximod), IDO1 inhibitors (e.g. Indoximod), or other immunomodulatory drugs, e.g. anti-inflammatory VIK peptide, curcuminoids, gingerol, silibinin, or similar phytochemicals (Pavlicevic et al., Peptides 148 (2022), and immunomodulatory drugs described in chapters 4 and 5 of Behl, et al., Exploring the multifocal role of phytochemicals as immunomodulators, Biomedicine & Pharmacotherapy, 133, 2021).

**TLR7/8**-Agonists (herein abbreviated as TLR7/8_ag) are usually used as adjuvant in cancer vaccination.

**Cucurbitacin B** (CuB) is a natural substance acting as immunomodulator and cytostatic at low concentration in nanomolar range, i.a. via JAK/STAT inhibition, and as cytotoxic agent at higher concentration in micromolar range, via cytoskeleton interruption.

**Benzylamine** can be used as a decoy cargo for the HSA nanocarrier system, which shows no biological effect in the applied concentration but still allowed for the loading procedure of the HSA since this alters its 3D-structure significantly. The in vitro data even indicates slight differences in uptake efficiency depending on the cargo that was loaded.

The term **"molar ratio immunomodulatory drug:HSA"** or synonymously "molar immunomodulatory drug:HSA ratio" refers to the number of molecules of immunomodulatory drug loaded on one HSA molecule. The "molar ratio immunomodulatory drug:HSA" depends on different factors such as the chemical structure and size of the immunomodulatory drug, and the resulting loading of the produced nanocarrier system.

In one embodiment, the inventive HSA nanocarrier system comprises one immunomodulatory drug. Thus, the present invention is directed to an HSA nanocarrier system for drug delivery into a target cell, wherein said nanocarrier system comprises:
A) a human serum albumin (HSA);
B) a plurality of mannose-based targeting structures covalently bound to said HSA;
C) an immunomodulatory drug covalently bound to said HSA;
wherein said HSA nanocarrier system has a diameter in the range comprised between 4 nm and 150 nm, as measured by dynamic light scattering.

Preferably, the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

In one embodiment, the inventive HSA nanocarrier system comprises two or more different immunomodulatory drugs. Thus, the present invention is directed to an HSA nanocarrier system for drug delivery into a target cell, wherein said nanocarrier system comprises:
A) a human serum albumin (HSA);
B) a plurality of mannose-based targeting structures covalently bound to said HSA; and
C) two or more immunomodulatory drugs covalently bound to said HSA;
wherein said HSA nanocarrier system has a diameter in the range comprised between 4 nm and 150 nm, as measured by dynamic light scattering.

Preferably, the two or more immunomodulatory drugs are selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

### Loading of drug to the nanocarrier system

In order to link the immunomodulatory drug of choice to HSA, the drug is preferably coupled to a drug linker that is capable of forming a covalent bond with a reactive group (e.g. amino group, thiol group, hydroxyl group, carboxy group etc.) present on the surface of (denatured) mannosylated HSA.

The term **"drug linker"** as used herein encompasses molecular fragments capable of connecting the immunomodulatory drug with HSA of mannosylated HSA, optionally by binding to at least one interconnecting molecule. Thus, the function of the drug linker *per se* or together with the interconnecting molecule is to establish, keep and/or bridge a special distance between the immunomodulatory drug and the HSA of mannosylated HSA. The drug linker is not capable of forming a covalent (or any other type of bond) bond with the mannose-based targeting structure of the mannosylated HSA. More specifically, one extremity of the drug linker is connected to the immunomodulatory drug and the other extremity is connected *via* a functional group capable of forming a covalent bond with a reactive group (e.g. amino group, thiol group, hydroxyl group, carboxy group etc.) present on the mannosylated HSA.

Preferably, the drug linker is functionalized with a **"thiol-reactive functional group"** reacting with free thiols on mannosylated HSA, such that the immunomodulatory drug is covalently bound to a thiol group on mannosylated HSA via the drug linker. Preferred **"thiol-reactive functional groups"** are selected from the group consisting of pyridinyldithiol-ethoxy-carbonyl-group, maleimide, mc-vc-pabc linker (maleimidocaproyl (mc) - valine-citrulline (vc)-para-amino benzyloxycarbonyl (PABC)), thiosulfonate linker (via disulfide exchange), hydrazine-maleimide linker if the drug has a ketone or aldehyde function, iodoacteamides, MTS reagents (MethaneThioSulfonate).

The term **"traceless drug linker"** or **"self-immolative drug linker"** as used herein refers to a drug linker comprising a bifunctional chemical moiety, which is capable of covalently linking together the immunomodulatory drug with HSA in spaced manner into a normally stable nanocarrier system, but which becomes labile upon activation (e.g., nucleophilic attack, protease) leading to rapid cleavage of the moiety and release of the immunomodulatory drug from the mannosylated HSA in its active form. In other words, when the traceless drug linker is activated by a specific stimulus, it undergoes spontaneous intramolecular disassembling, in which it is broken down to its building blocks, and the immunomodulatory drug and mannosylated HSA are released, each without a remaining fragment of the linker.

Preferred "traceless drug linkers" are selected from the group consisting of pyridinyldithiol-ethoxy-carbonyl, and mc-vc-pabc linker.

The traceless PABC drug linker allows the release of the immunomodulatory drug containing at least one amine, phenol, carboxylic acid, or thiol functionality upon cleavage of the protease-cleavable substrate by endogenous or exogenous proteases and 1,6-spontaneous fragmentation. Cleavage kinetics is independent of the identity of released amine, phenol, carboxylic acid, or thiol molecules. Moreover, PABC can enhance the cleavage rate due to the presence of the p-aminobenzyl group.

**Table 1 Summary of schematic symbols used herein and with the corresponding description and coding**

| **Structure** | **Description** | **Codes** |
|---|---|---|
| **A** | native unmodified HSA purchased from sigma Aldrich | HSA |
| **B** | dye (Sulfo-Cy5 or Sulfo-Cy7.5) labeled HSA | ^{Cy5/7.5}HSA |
| **C** | dye (sulfo-Cy5 or sulfo-Cy7.5) labeled HSA with ring-opened maleimide | ^{Cy5/7.5}HSA |
| **D** | dye-labeled HSA with DBCO groups attached to the surface | ^{Cy5/7.5}HSA-(DBCO)ₙ, ^{Cy5/7.5}HSA-(DBCO)ₙ, (DBCO)ₙ-^{Cy5/7.5}HSA, DBCO-^{Cy5/7.5}HSA |
| **E** | dye-labeled HSA with TM groups clicked to the DBCO on the surface (dye-labeled mannosylated HSA) | ^{Cy5/7.5}HSA-(TM)ₙ, TM-^{Cy5/7.5}HSA, TM-^{Cy5/7.5}HSA |
| **F** | dye-labeled HSA with TM on the surface which was completely denatured, decoy-loaded and backfolded (different structure). | ^{Cy5/7.5}HSA-(TM)ₙ-Benzyl, (TM)ₙ-^{Cy5/7.5}HSA-Benzyl, TM-^{Cy5/7.5}HSA-Benzyl |
| **G** | dye-labeled HSA with TM on the surface which was completely denatured, TLR7/8-agonist-loaded and backfolded (different structure). (nanocarrier system) | ^{Cy5/7.5}HSA-(TM)ₙ-TLR7/8, (TM)ₙ-^{Cy5/7.5}HSA-TLR7/8, TM-^{Cy5/7.5}HSA-TLR7/8, ^{Cy5/7.5}HSA-(TM)ₙ-TLR7/8_ag, (TM)ₙ-^{Cy5/7.5}HSA-TLR7/8_ag, TM-^{Cy5/7.5}HSA-TLR7/8_ag |
| **H** | dye-labeled HSA with TM on the surface which was completely denatured, CuB-loaded and backfolded (different structure). (nanocarrier system) | ^{Cy5/7.5}HSA-(TM)ₙ-CuB, (TM)ₙ-^{Cy5/7.5}HSA-CuB, TM-^{Cy5/7.5}HSA-CuB |
| **I** | trimannose | TM |
| **J** | dye (e.g. Sulfo-Cy5 or Sulfo-Cy7.5) | Cy5, Cy7.5 |
| **K** | Immunomodulatory drug (e.g. Curcubitacin B, TLR7/8 agonist) | CuB, TLR7/8, TLR7/8_ag (R848) |

Thus, the present invention is directed to an HSA nanocarrier system for drug delivery into a target cell, wherein said nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering.

Preferably, the HSA nanocarrier system has a diameter in the range comprised between 4 nm and 100 nm. More preferably, the HSA nanocarrier system has a diameter in the range comprised between 4 nm and 20 nm.

### Pharmaceutical compositions

**"Pharmaceutical composition"** refers to a preparation in a form that allows the biological activity of the active ingredient(s) to be effective, and which contain no additional components which are toxic to the subjects to which the formulation is administered.

As used herein, the terms **"pharmaceutically acceptable", "physiologically tolerable"** and grammatical variations thereof, are used interchangeably and represent that the materials are capable of administration to or upon a mammal without the production of undesirable physiological effects such as nausea, dizziness, gastric upset and the like.

The pharmaceutical composition is designed to facilitate the administering of the inventive HSA nanocarrier system in an effective manner.

**"Pharmaceutically acceptable excipient"** refers to an ingredient in a pharmaceutical formulation, other than an active ingredient, which is nontoxic to the subject to whom it is administered. A pharmaceutically acceptable excipient includes, but is not limited to, a buffer, stabilizer, or preservative.

Examples of suitable excipients include, without limitation, lactose, dextrose, sucrose, glucose, powdered sugar, sorbitol, mannitol, xylitol, starches, acacia gum, xanthan gum, guar gum, tara gum, mesquite gum, fenugreek gum, locust bean gum, ghatti gum, tragacanth gum, inositol, molasses, maltodextrin, extract of Irish moss, panwar gum, mucilage of isapol husks, Veegum, larch arabogalactan, calcium silicate, calcium phosphate, dicalcium phosphate, calcium sulfate, kaolin, sodium chloride, polyethylene glycol, alginates, gelatine, microcrystalline cellulose, polyvinylpyrrolidone, cellulose, water, saline, syrup, methylcellulose, ethylcellulose, hydroxypropylnethylcellulose, carboxymethylcellulose, polyacrylic acids such as Carbopols, such as Carbopol941, Carbopol980, Carbopol981,and gum bases such as Pharmagum^{™} (SPI Pharma Group; New Castle, Del.), and similar. Typically, the compositions of the present invention comprise from about 10% to about 90% by weight of the excipient or combinations thereof.

Preferably, the pharmaceutical composition contains from about 0.001% to about 90%, preferably from about 0.01% to about 75%, more preferably from about 0.1% to 50%, and still more preferably from about 0.1% to 10% by weight of the HSA nanocarrier system of the present invention, with the remainder consisting of suitable pharmaceutical excipients, and/or diluents.

The pharmaceutical composition can be formulated into powders, granules, tablets, capsules, suspensions, emulsions, syrups, oral dosage form, external preparation, suppository or in the form of sterile injectable solutions, such as aerosolized in a usual manner, respectively. When formulated, it can be prepared using a **diluent** or **excipient** such as generally used fillers, extenders, binders, wetting agents, disintegrating agents, surface active agents.

In the pharmaceutical composition, the solid preparation for oral administration may be a tablet, pill, powder, granule, or capsule. The solid preparation may further comprise an excipient. Excipients may be, for example, starch, calcium carbonate, sucrose, lactose, or gelatine. In addition, the solid preparation may further comprise a lubricant, such as magnesium stearate, or talc. In the pharmaceutical composition, liquid preparations for oral administration may be best suspensions, solutions, emulsions, or syrups. The liquid formulation may comprise water, or liquid paraffin. The liquid formulation may, for excipients, for example, include wetting agents, sweeteners, aromatics or preservatives. For the purposes of parenteral administration, compositions containing the HSA nanocarrier system of the invention are preferably dissolved in distilled water and the pH preferably adjusted to about 6 to 8.

Useful preparations of the compositions of the invention for parenteral administration also include sterile aqueous and non-aqueous solvents, suspensions and emulsions. Examples of useful non-aqueous solvents include propylene glycol, polyethylene glycol, vegetable oil, fish oil, and injectable organic esters.

An embodiment of the present invention is directed to a pharmaceutical composition comprising an HSA nanocarrier system for drug delivery into a target cell and a pharmaceutically acceptable excipient and/or diluent, wherein said nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering.

Another embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the HSA bound to the plurality of mannose-based targeting structures and to the at least one immunomodulatory drug is backfolded by a rapid dilution step.

Another embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the mannose-based targeting structure of the plurality of mannose-based targeting structures is selected from the group consisting of Man*p*N₃, ManpsNs, (Man*p*)*₃*N₃, and (Man*p₃*)₃N₃.

Most preferably, the mannose-based targeting structure is Man*p₃*N₃ (trimannose).

Preferably, the pharmaceutical composition comprises a HSA nanocarrier system for drug delivery into a target cell and a pharmaceutically acceptable excipient and/or diluent, wherein the nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

Another embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the molar immunomodulatory drug:HSA ratio is comprised between 1 and 30

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the molar immunomodulatory drug:HSA ratio is comprised between 1 and 30, and wherein the mannose-based targeting structure of the plurality of mannose-based targeting structures is selected from the group consisting of Man*p*N₃, Man*p*₃N₃, (Man*p*)*₃*N₃.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the molar immunomodulatory drug:HSA ratio is comprised between 1 and 30, and wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

Another embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the molar mannose-based targeting structure:HSA ratio is comprised between 1 and 59.

Another embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the plurality of mannose-based targeting structures is covalently bound to the HSA via an interconnecting molecule and the molar ratio of interconnecting molecule:HSA is comprised between 3 and 59, and wherein the molar mannose-based targeting structure:HSA ratio is comprised between 1 and 59.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the plurality of mannose-based targeting structures is covalently bound to the HSA via an interconnecting molecule, and wherein the molar mannose-based targeting structure:HSA ratio is comprised between 1 and 59.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the plurality of mannose-based targeting structures is covalently bound to the HSA via an interconnecting molecule and the molar ratio of interconnecting molecule:HSA is comprised between 3 and 59, wherein the molar mannose-based targeting structure:HSA ratio is comprised between 1 and 59, wherein the molar immunomodulatory drug:HSA ratio is comprised between 1 and 30, and wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the plurality of mannose-based targeting structures is covalently bound to the HSA via an interconnecting molecule and the molar ratio of interconnecting molecule:HSA is comprised between 3 and 59, wherein the molar mannose-based targeting structure:HSA ratio is comprised between 1 and 59, wherein the molar immunomodulatory drug:HSA ratio is comprised between 1 and 30, and wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

Another embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, and wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, and wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

Another embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the immunomodulatory drug is covalently bound to said HSA by a drug linker using a thiol-reactive functional group selected from the group consisting of maleimide, iodoacetamide, and iodoacetate.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a drug linker using a thiol-reactive functional group selected from the group consisting of maleimide, iodoacetamide, and iodoacetate, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a drug linker using a thiol-reactive functional group selected from the group consisting of maleimide, iodoacetamide, and iodoacetate, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a drug linker using a thiol-reactive functional group selected from the group consisting of maleimide, iodoacetamide, and iodoacetate, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a drug linker using a thiol-reactive functional group selected from the group consisting of maleimide, iodoacetamide, and iodoacetate, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a drug linker using a thiol-reactive functional group selected from the group consisting of maleimide, iodoacetamide, and iodoacetate, wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, wherein the mannose-based targeting structure of the plurality of mannose-based targeting structures is selected from the group consisting of Man*p*N₃, Man*p*₃N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

Another embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the immunomodulatory drug is covalently bound to said HSA by a traceless, self-immolative drug linker comprising a thiol-reactive functional group, wherein the traceless, self-immolative linker is selected pyridinyldithiol-ethoxy-carbonyl or mc-vc-pabc linker (maleimidocaproyl (mc) - valine-citrulline (vc) -para-amino benzyloxycarbonyl (PABC)).

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a traceless, self-immolative drug linker comprising a thiol-reactive functional group, wherein the traceless, self-immolative linker is covalently bound to a ketone or aldehyde group of the immunomodulatory drug and the linker is selected from methanethiosulfonate, thiosulfonate, and maleimide-hydrazide (e.g. BMPH (N-β-maleimidopropionic acid hydrazide)), wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, wherein the mannose-based targeting structure of the plurality of mannose-based targeting structures is selected from the group consisting of Man*p*N₃, Man*p₃*N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a traceless, self-immolative drug linker comprising a thiol-reactive functional group, wherein the traceless, self-immolative linker is covalently bound to a ketone or aldehyde group of the immunomodulatory drug and the linker is selected from methanethiosulfonate, thiosulfonate, and maleimide-hydrazide (e.g. BMPH (N-β-maleimidopropionic acid hydrazide)), and wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a traceless, self-immolative drug linker comprising a thiol-reactive functional group, wherein the traceless, self-immolative linker is covalently bound to a ketone or aldehyde group of the immunomodulatory drug and the linker is selected from methanethiosulfonate, thiosulfonate, and maleimide-hydrazide (e.g. BMPH (N-β-maleimidopropionic acid hydrazide)), wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein the immunomodulatory drug is covalently bound to said HSA by a traceless, self-immolative drug linker comprising a thiol-reactive functional group, wherein the traceless, self-immolative linker is covalently bound to a ketone or aldehyde group of the immunomodulatory drug and the linker is selected from methanethiosulfonate, thiosulfonate, and maleimide-hydrazide (e.g. BMPH (N-β-maleimidopropionic acid hydrazide)), wherein the immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin, wherein the plurality of mannose-based targeting structures is covalently bound to the HSA via an interconnecting molecule and the molar ratio of interconnecting molecule:HSA is comprised between 3 and 59, and/or wherein the molar mannose-based targeting structure:HSA ratio is comprised between 1 and 59, wherein the molar immunomodulatory drug:HSA ratio is comprised between 1 and 30, wherein the mannose-based targeting structure of the plurality of mannose-based targeting structures is selected from the group consisting of Man*p*N₃, Man*p₃*N₃, (Man*p*)*₃*N₃, and (Man*p₃*)₃N₃ and wherein the HSA bound to the plurality of mannose-based targeting structures and to the at least one immunomodulatory drug is backfolded by a rapid dilution step.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, wherein HSA is linked to the mannose-based targeting structure by an azide-reactive interconnecting molecule selected from the group consisting of DBCO, Endo-BCN, DIBO, DIFO, BCN.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
D) a fluorescent dye covalently bound to said HSA, wherein the fluorescent dye comprises a maleimide as thiol-reactive functional group or an NHS-ester as amine-reactive functional group, and wherein the fluorescent dye emits light in a range of 400 nm to 850 nm.
wherein said HSA nanocarrier system has a diameter in the range comprised between 4 nm and 150 nm, as measured by dynamic light scattering.

Another preferred embodiment of the present invention is directed to a pharmaceutical composition comprising an HSA nanocarrier system for drug delivery into a target cell and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
D) a fluorescent dye covalently bound to said HSA, wherein the fluorescent dye comprises a maleimide as thiol-reactive functional group, and wherein the fluorescent dye emits light in a range of 400 nm to 850 nm.

wherein said HSA nanocarrier system has a diameter in the range comprised between 4 nm and 150 nm, as measured by dynamic light scattering, and
wherein the fluorescent dye is selected from the group consisting of sulfo-cyanin-maleimide, Alexa Fluor-maleimide, and cyanin-maleimide.

Another embodiment of the present invention is directed to a pharmaceutical composition comprising an HSA nanocarrier system for drug delivery into a target cell expressing a mannose surface receptor and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering.

Another embodiment of the present invention is directed to a pharmaceutical composition comprising an HSA nanocarrier system for drug delivery into a target cell expressing a mannose surface receptor and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the target cells express a mannose surface receptor selected from the group consisting of CD206, CD209, and homologues thereof.

Another embodiment of the present invention is directed to a pharmaceutical composition comprising an HSA nanocarrier system for drug delivery into a target cell expressing a mannose surface receptor and a pharmaceutically acceptable excipient and/or diluent, wherein the HSA nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering, and wherein the target cells are selected from the group consisting of dendritic cells, macrophages, B cells, T cells, liver non-parenchymal cells, splenic cells, neutrophils, and cultured cells expressing an exogenous surface mannose receptor.

Another preferred embodiment of the present invention is directed to a pharmaceutical composition comprising an HSA nanocarrier system for drug delivery into a target cell expressing a mannose surface receptor and a pharmaceutically acceptable excipient and/or diluent, wherein the HSA nanocarrier system comprises:
- trimannose bound via a NHS-PEGn-DBCO interconnecting molecule to HSA at a molar trimannose:HSA range comprised between 1 and 59;
- Cucurbitacin B or TLR7/8 agonist as an immunomodulatory drug at a molar drug:HSA range preferably comprised between 5 and 27, wherein the immunomodulatory drug is covalently bound to said HSA by a traceless, self-immolative pyridinyldithio-ethoxy-carbonyl drug linker;
- optionally a sulfo-Cyanin5-maleimide dye or a sulfo-Cyanin7.5-maleimide dye conjugated to said HSA
and wherein said HSA nanocarrier system has a diameter in the range comprised between 4 nm and 150 nm.

### Preparation of HSA-nanocarrier systems

Another aspect of the present invention is directed to a method to manufacture a HSA nanocarrier system as described herein, wherein the method comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system,
wherein the HSA nanocarrier system comprises:
   A) a human serum albumin (HSA);
   B) a plurality of mannose-based targeting structures covalently bound to said HSA;
   C) at least one immunomodulatory drug covalently bound to said HSA;
wherein said HSA nanocarrier system has a diameter in the range comprised between 4 nm and 150 nm, as measured by dynamic light scattering.

In step iv. of the method to manufacture the inventive HSA-nanocarrier system, denaturing of the mannosylated HSA results in the release of thiol groups by reduction of native disulfides to which in the later steps the immunomodulatory drug is covalently bound. Preferably, the mannosylated HSA is completely denatured in step iv.

In step v.-a of the method to manufacture the inventive HSA-nanocarrier system, the at least one immunomodulatory drug is preferably covalently bound to the mannosylated HSA via a disulfide exchange reaction. The free thiols of HSA resulting from the denaturation step react with a thiol-reactive functional group (such as pyridinyldithio-ethoxy-carbonyl-group or maleimide) attached to the at least one immunomodulatory drug.

In step vi. of the method to manufacture the inventive HSA-nanocarrier system, the backfolding is preferably carried out such that not the exact native conformation of HSA is obtained, but an α-helix-based globular structure of the monomolecular HSA nanocarrier system

In one embodiment, the inventive HSA-nanocarrier system comprises one immunomodulatory drug. Thus, the method to manufacture a HSA nanocarrier system as described herein, comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of an immunomodulatory drug to said denatured mannosylated HSA, wherein the immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system,
wherein the HSA nanocarrier system comprises:
   A) a human serum albumin (HSA);
   B) a plurality of mannose-based targeting structures covalently bound to said HSA;
   C) at least one immunomodulatory drug covalently bound to said HSA;
wherein said HSA nanocarrier system has a diameter in the range comprised between 4 nm and 150 nm, as measured by dynamic light scattering.

In one embodiment, the inventive HSA-nanocarrier system comprises at least two immunomodulatory drugs. Thus, the method to manufacture a HSA nanocarrier system as described herein, comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a1) covalently binding of an immunomodulatory drug to said denatured mannosylated HSA, wherein the immunomodulatory drug is attached to a drug linker comprising a thiol-reactive functional group;
v.-a2) repeating step v.-a1 with another immunomodulatory drug of the at least two immunomodulatory drugs, until all immunomodulatory drugs are covalently bound to the denatured mannosylated HSA;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system
wherein the HSA nanocarrier system comprises:
   A) a human serum albumin (HSA);
   B) a plurality of mannose-based targeting structures covalently bound to said HSA;
   C) at least one immunomodulatory drug covalently bound to said HSA;
wherein said HSA nanocarrier system has a diameter in the range comprised between 4 nm and 150 nm, as measured by dynamic light scattering.

In a preferred embodiment of method to manufacture a HSA nanocarrier system as described herein, the method comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system,
wherein the monomolecular HSA nanocarrier system has a molar mannose:HSA ratio comprised between 1 and 59, and/or a molar interconnecting molecule:HSA ratio comprised between 3 and 59.

In a preferred embodiment of method to manufacture a HSA nanocarrier system as described herein, the method comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system,
wherein the mannose-based targeting structure is selected from the group consisting of Man*p*N₃, Man*p*₃N₃, (Man*p*)*₃*N₃, and (Man*p₃*)₃N₃.

In a preferred embodiment of method to manufacture a HSA nanocarrier system as described herein, the method comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system,
wherein the at least one immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

In a preferred embodiment of method to manufacture a HSA nanocarrier system as described herein, the method comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system,
wherein the molar immunomodulatory drug:HSA ratio is comprised between 1 and 30.

In a preferred embodiment of method to manufacture a HSA nanocarrier system as described herein, the method comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system,
wherein the at least one immunomodulatory drug is covalently bound to said HSA by a traceless, self-immolative linker comprising a thiol-reactive functional group.

In a preferred embodiment of method to manufacture a HSA nanocarrier system as described herein, the method comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system,
wherein the drug linker is selected from the group consisting of pyridinyldithio-ethoxy-carbonyl-group, maleimide, mc-vc-pabc linker, thiosulfonate linker, hydrazide-maleimide linker, iodoacetamide linker, methanethiosulfonate linker.

In a preferred embodiment of method to manufacture a HSA nanocarrier system as described herein, the method comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system,
wherein the interconnecting molecule in step ii. comprises an azide-reactive group selected from DBCO, Endo-BCN, DIBO, DIFO, and BCN.

In a preferred embodiment of method to manufacture a HSA nanocarrier system as described herein, the method comprises the following steps:
i.) providing monomolecular native HSA, wherein the monomolecular native HSA is labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system,
wherein the HSA is labeled with a fluorescent dye emitting fluorescence in a range comprised between 400 and 850 nm.

In a preferred embodiment of method to manufacture a HSA nanocarrier system as described herein, the method comprises the following steps:
i.) providing monomolecular native HSA, wherein the monomolecular native HSA is labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system,
wherein the HSA is labeled with a fluorescent dye via a maleimide or an NHS-ester.

In a preferred embodiment of method to manufacture a HSA nanocarrier system as described herein, the method comprises the following steps:
i.) providing monomolecular native HSA, wherein the monomolecular native HSA is labeled with Sulfo Cy7.5-NHS via an NHS-ester;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system.

In a preferred embodiment, the method to manufacture a HSA nanocarrier system as described herein, comprises the following steps:
i.) providing monomolecular native HSA, wherein the monomolecular native HSA is optionally labeled with a fluorescent dye;
i.1) subjecting the HSA labeled with a fluorescent dye maleimide to a ring-opening hydrolysis by adding a buffer at a pH above 9.
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drug to said denatured mannosylated HSA, wherein the at least one immunomodulatory drug is attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system.

### Description of the Figures

- **Figure 1**: shows in A) a general structure of the inventive mannose-HSA nanocarrier system. B) shows the chemical structure of mannose-based targeting structures as disclosed herein.

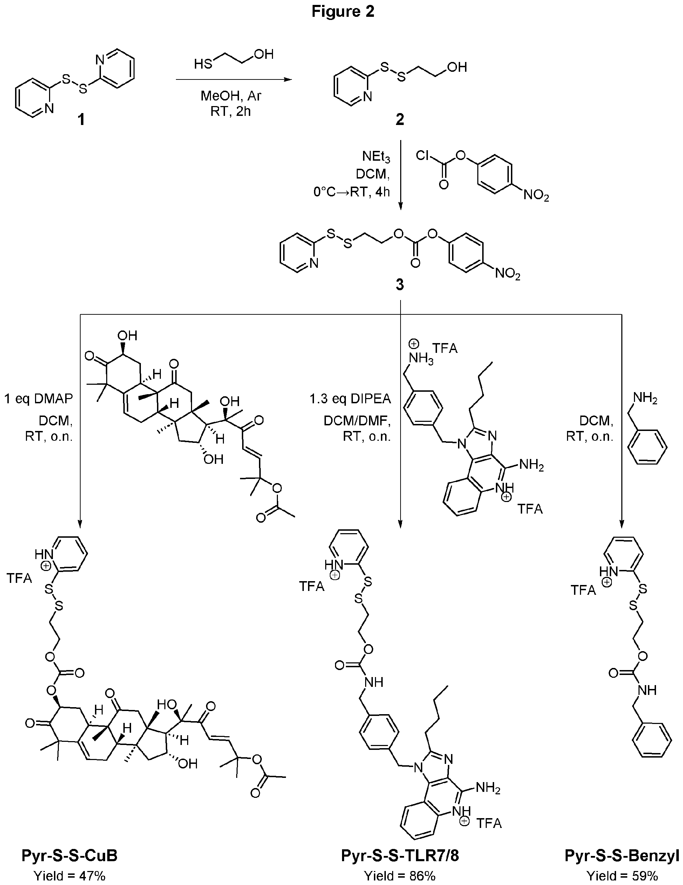

- **Figure 2**: shows synthesis scheme for"Pyr-S-S-TLR7/8_ag", "Pyr-S-S-CuB", and "Pyr-S-S-Benzyl".

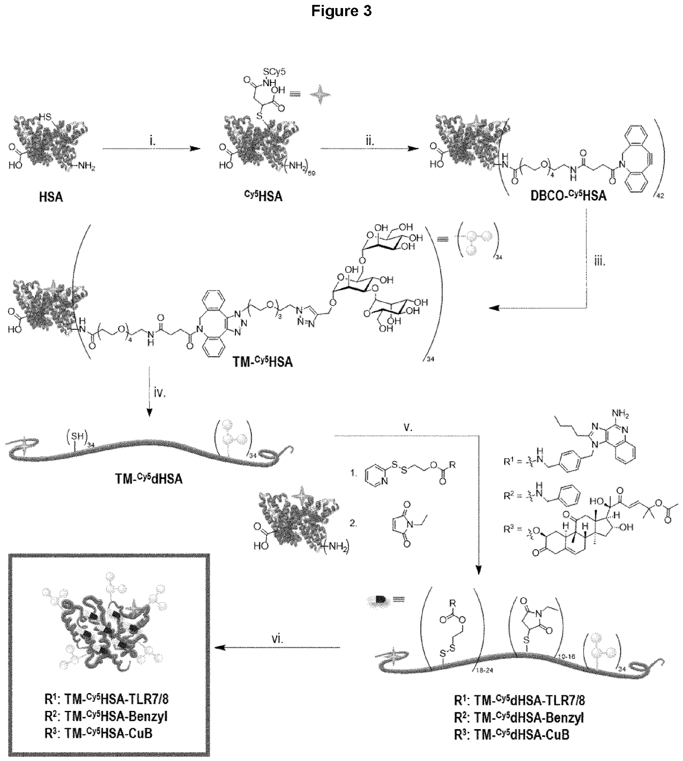

- **Figure 3**: shows synthesis scheme of trimannosylated HSA. **i.)** 50 mM PB pH 7.4, RT, 4 h; 50 mM Na-borate buffer pH 9.2, 37 °C, o.n. **ii.)** NHS-PEG₄-DBCO, 50 mM PB pH 7.4, RT, 4-6h **iii.)** 50 mM PB (8 M urea, 2 mM EDTA) pH 8, RT, 4-16h **iv.)** TCEP, 50 mM PB (8 M urea, 2 mM EDTA) pH 8 **v.)** 1. Pyr-S-S-R, 2. N-Ethylmaleimide; 50 mM PB (8 M urea, 2 mM EDTA) pH 8 **vi.)** rapid dilution, MQ. HSA crystal structure has been described by Sugio, S., et al. 1999. Crystal structure of human serum albumin at 2.5 A resolution. Protein Eng 12: 439-446.

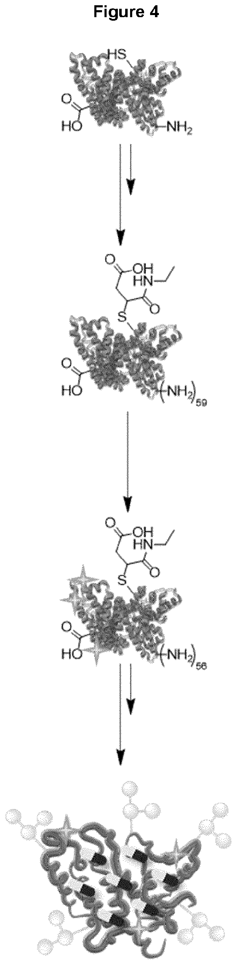

- **Figure 4**: shows the differing steps of the synthesis of the TM-HSA-nanocarrier system as described herein for in vivo experiments (description in Example 1).

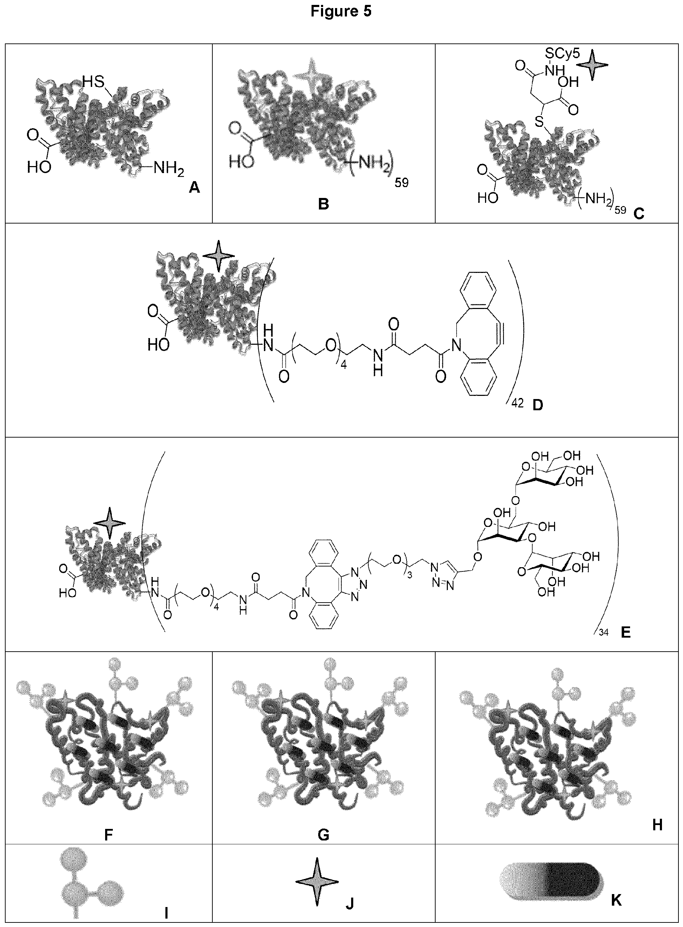

- **Figure 5**: shows an overview of the schematic symbols used herein to indicate the different HSA conjugates. A) HSA; B) dye (sulfo-Cy5 or sulfo-Cy7.5) labeled HSA; C) dye (sulfo-Cy5 or sulfo-Cy7.5) labeled HSA with ring-opened maleimide; D) dye-labeled HSA with DBCO groups attached to the surface; E) dye-labeled trimannosylated HSA, wherein the trimannose is clicked to the DBCO on the surface; F) dye-labeled TM-HSA nanocarrier system, which was completely denatured, decoy-loaded or TLR7/8_Agonist-loaded or CuB-loaded and backfolded (different structure); G) dye-labeled HSA with TM on the surface which was completely denatured, TLR7/8-agonist-loaded and backfolded (different structure); H) dye-labeled HSA with TM on the surface which was completely denatured, CuB-loaded and backfolded (different structure); I) trimannose; J) dye (e.g. Sulfo-Cy5 or Sulfo-Cy7.5); K) Immunomodulatory drug (e.g. Curcubitacin B, TLR7/8 agonist).

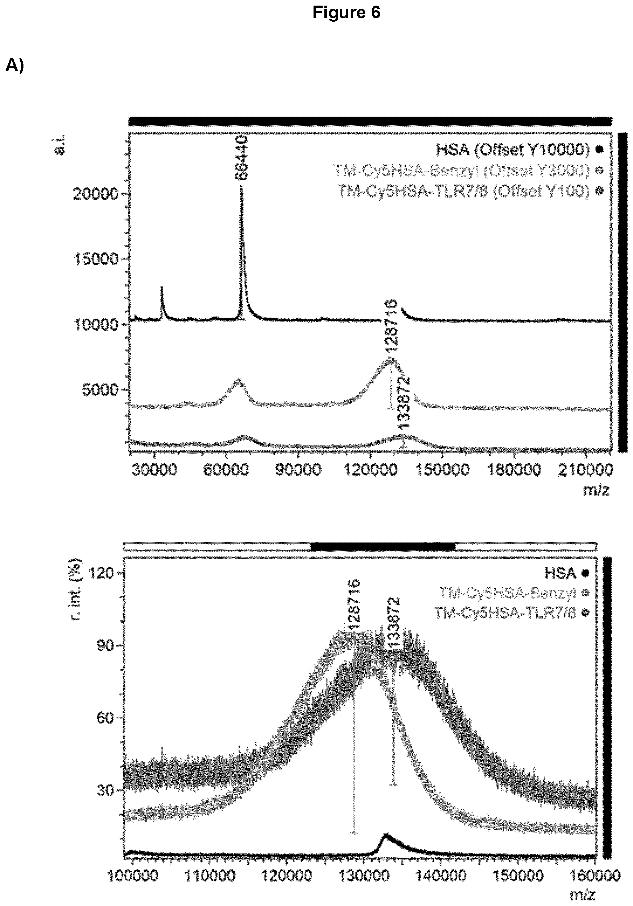

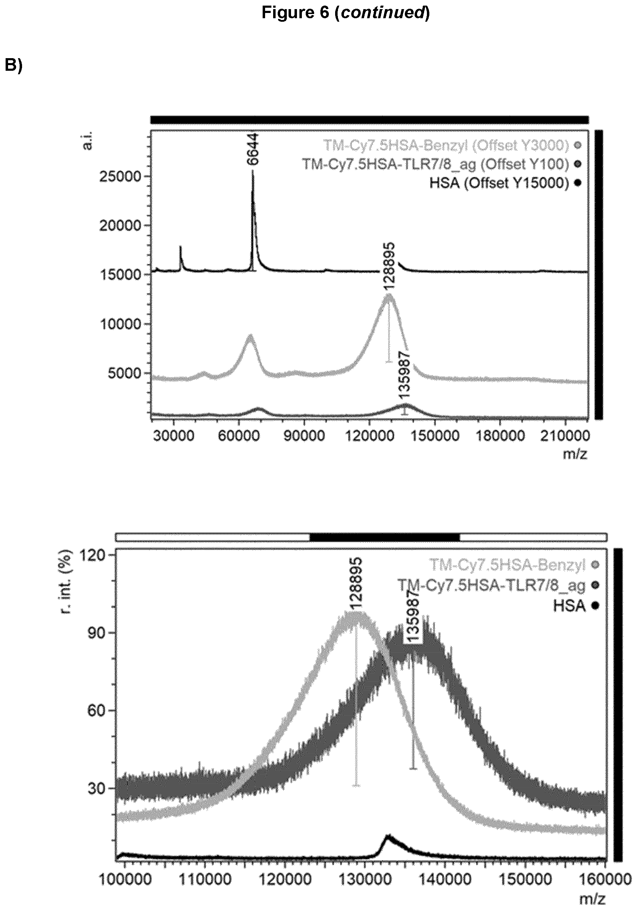

- **Figure 6**: shows characterization of loaded TM-HSA-nanocarrier systems by MALDI-ToF mass spectrometry compared to HSA (A,B; full spectra left, region of interest right).
- **Figure 7**: shows characterization of benzyl-loaded TM-HSA-nanocarrier system by fluorescence correlation spectroscopy yielding a calculated hydrodynamic radius of 12 nm in water and 15 nm in blood plasma.

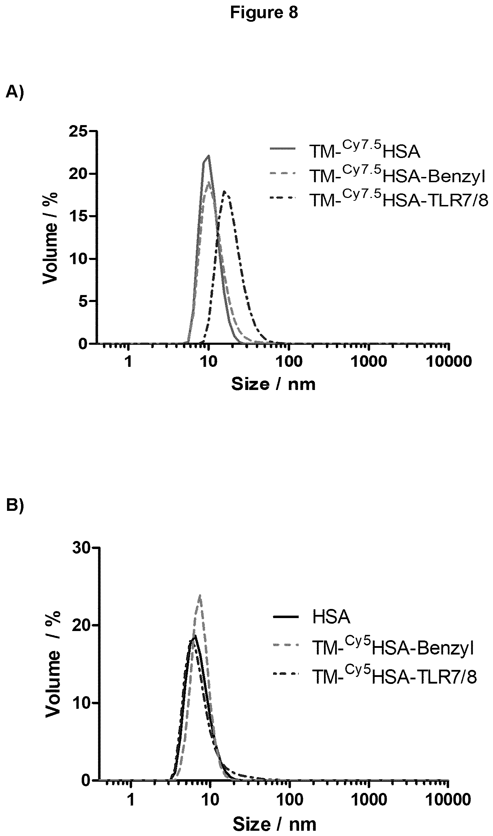

- **Figure 8**: shows characterization of loaded TM-HSA- nanocarrier systems (1 mg/mL) compared to HSA or unloaded trimannosylated HSA by dynamic light scattering analysis (DLS) in A) PBS and B) MQ. Volume distribution was used to determine the sizes of HSA (d.nm. = 7.37 ± 2.46), TM-^{Cy5}HSA-Benzyl (d.nm = 9.51 ± 2.76), TM-^{Cy5}HSA-TLR7/8_ag (d.nm = 8.11 ± 7.71), TM-^{Cy7.5}HSA-TM (d.nm = 10.71 ± 2.85), TM-^{Cy7.5}HSA-Benzyl (d.nm = 12.73 ± 9.75), TM-^{Cy7.5}HSA-TLR7/8_ag (d.nm = 19.94 ± 7.62).

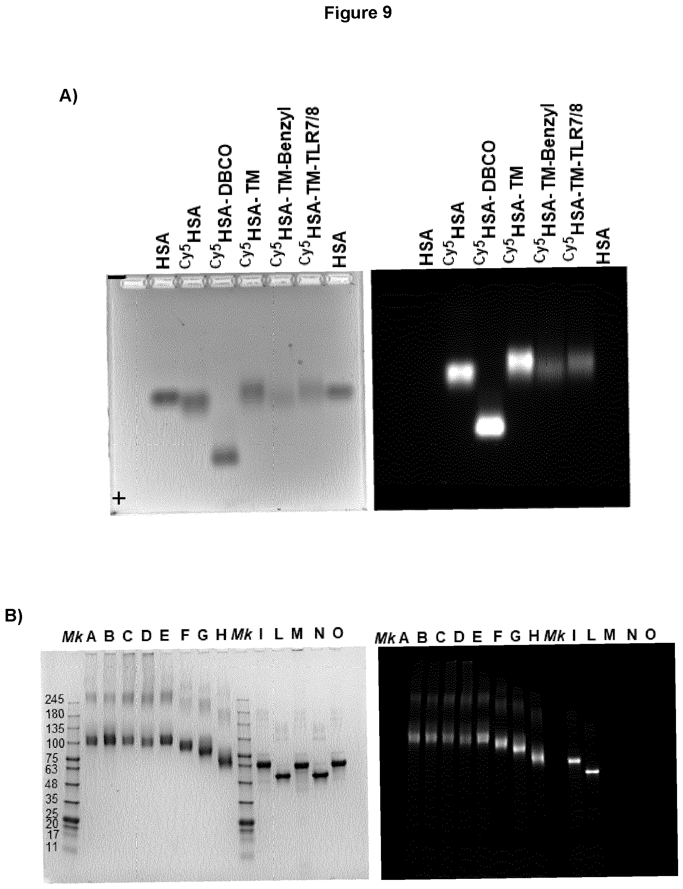

- **Figure 9**: shows gel electrophoresis characterization (size & charge) of loaded TM-HSA-nanocarrier systems. A) Agarose gel electrophoresis; B) SDS-PAGE. Loaded samples: Mk = Marker; A = TM- ^{Cy5}HSA-TLR7/8_{Red}; B = TM- ^{Cy5}HSA-TLR7/8_{NonRed}; C = TM- ^{Cy5}HSA-Benzyl_{Red}; D = TM- ^{Cy5}HSA-Benzyl_{NonRed}; E = TM- ^{Cy5}HSA_{Red}; F = TM- ^{Cy5}HSA_{NonRed}; G = DBCO-^{Cy5}HSA_{Red}; H = DBCO- ^{Cy5}HSA_{NonRed}; Mk = Marker; I = ^{Cy5}HSA_{Red}; L = ^{Cy5}HSA_{NonRed}; M = HSA_{Red}; N = HSA_{NonRed}; O = HSA_{Red}; Red = reduced, NonRed = non-reduced.
- **Figure 10**: shows confocal microscopy images of CHO^{MMR+} cells treated for 24h with labeled ^{Cy5}HSA without trimannose and without cargo (CTRL, image "A"), or treated with ^{Cy5}HSA-(TM)₃₄ (images B1, C1, B2, C2) at a concentration of 1 µM. After treatment, CHO^{MMR+} cells were washed, fixed, stained and visualized at confocal microscope. Nuclei were stained with NucBlue. C1 and C2: z-stacks proof internalization rather than outer membrane attachment.

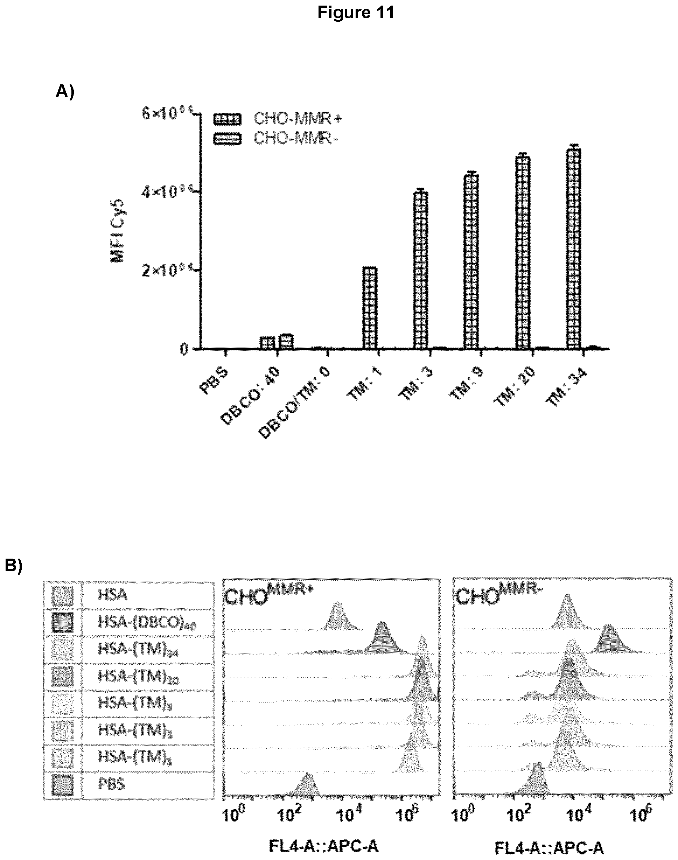

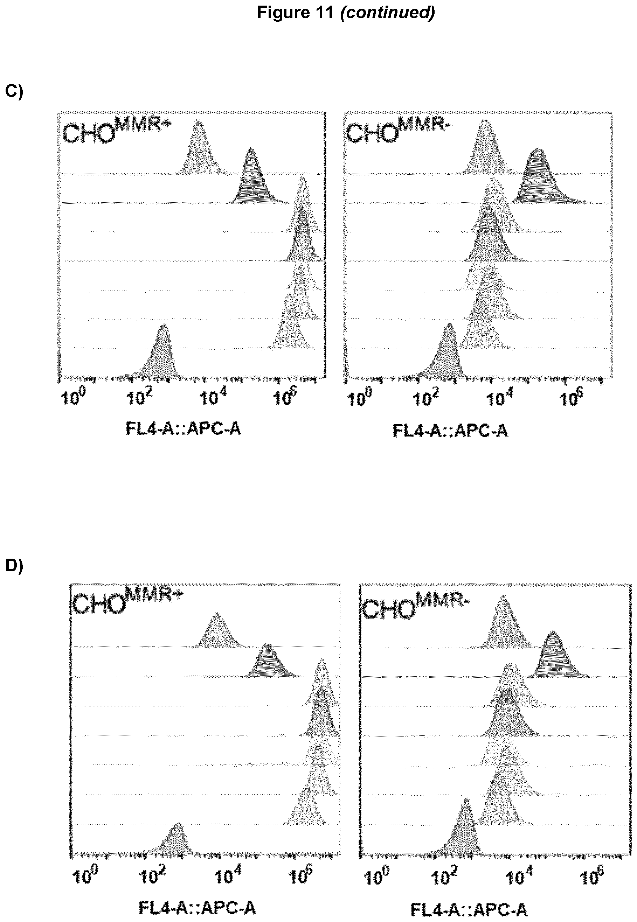

- **Figure 11**: shows FACS analysis of uptake of different HSA nanocarrier systems by CHO-K1 cells (MMR-), and transfected CHO^{MMR+.} Cells were treated for 24h with ^{Cy5}HSA "native" (=TM/DBCO:0), ^{Cy5}HSA-(DBCO)_{40.2} (=DBCO:40.2), ^{Cy5}HSA-(TM)_{34.1} (=TM: 34.1), ^{Cy5}HSA-(TM)_{20.1} (=TM: 20.1), ^{Cy5}HSA-(TM)_{9.2} (=TM: 9.2), ^{Cy5}HSA-(TM)_{2.9} (=TM: 2.9), ^{Cy5}HSA-(TM)_{1.3} (=TM: 1.3) at a concentration of 0.1 µM. The data of A) was obtained by three replicate experiments, each performed in triplicates; B)-D) show the stacked histograms for each experiment.

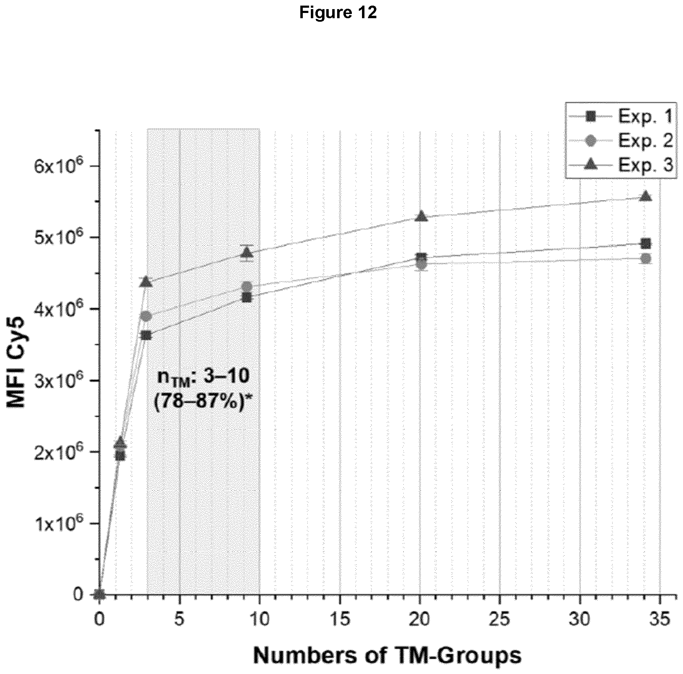

- **Figure 12**: shows uptake of different HSA nanocarrier systems by CHO^{MMR+} based on results of experiments presented in figure 11, on the basis of Cy5 fluorescence, in function of number of TM groups on HSA structure. For n(TM) between 3 and 10, the fluorescence was 78-87% of the maximal fluorescence reached.
- **Figure 13**: shows FACS analysis of uptake of different HSA nanocarrier systems by Liver NPC (A-C) and GM-CSF differentiated BMDC (D,E) as well as DC-SIGN expression in GM-CSF differentiated BMDC upon treatment with HSA nanocarrier systems (F). A-C: Liver NPC were treated with HSA nanocarrier systems having the TM amount indicated on graph axis overnight at 0.1 µM (n=1). D: BMDC were treated with HSA nanocarrier systems having the TM amount indicated on graph axis overnight at 0.1 µM (n=3). E: BMDC were treated with HSA nanocarrier systems having the TM amount indicated on graph axis overnight at 0.05, 0.1 and 0.25 µM (n=3). F: shows to E corresponding DC-expression upon treatment. Results show TM-dependent uptake in receptor positive cells. For conjugates with low-TM (1, 9), the uptake depends on the concentration of the nanocarrier in the culture medium. Differently, uptake saturation was observed with high TM (≥20) conjugates already at low nanocarrier concentration (0.05 µM). No significant change in DC-SIGN expression was observed in treated BMDC. BMDC: Bone marrow derived dendritic cells (D, E); DC: Dendritic cells from liver NPC (A); LSECs: Liver sinusoidal endothelial cells from liver NPC (B); Kupffer Cells from liver NPC: Liver Macrophages (C); DC-SIGN: Dendritic cell-specific intercellular adhesion molecule-3-grabbing non-integrin (F). A, B, D, E, F: Cy5 NP MFI; C: CD209a MFI.
- **Figure 14**: shows FACS analysis of uptake of different HSA nanocarrier systems by GM-CSF differentiated BMDC and immunostimulation after 3 h and overnight incubation time. A) Cy5 MFI to measure uptake; B) CD86 MFI; C) MHCII MFI; D) Cy5 MFI fold of CTRL; E) CD86 MFI fold of CTRL; F) MHCII MFI fold of CTRL. Cells were incubated with the different HSA nanocarriers at 50 nM, n=4-6.
- **Figure 15**: shows FACS analysis of uptake of different HSA nanocarrier systems by GM-CSF differentiated BMDC and immunostimulation after overnight incubation. A) Cy5 MFI; B) Cy5 MFI fold of CTRL; C) CD86 MFI; D) CD86 MFI fold of CTRL. ^{Cy5}HSA-TM-Benzyl [BL149] was tested also after preincubation with native and heat inactivated mouse serum for 30 min at 37 °C to get indications about protein corona formation which could impede uptake. Cells were incubated with the different HSA nanocarriers at 50 nM, n=3.

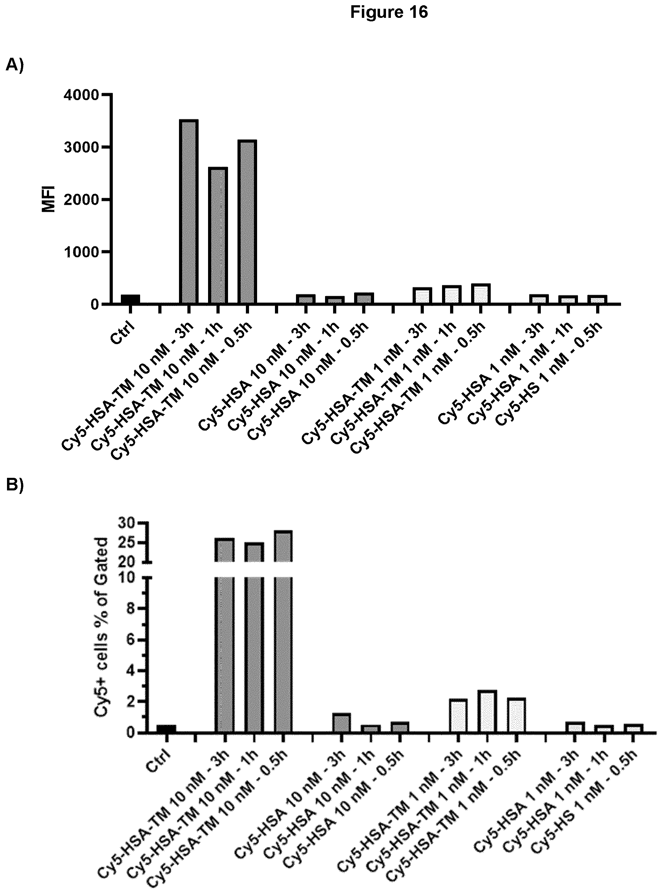

- **Figure 16**: shows FACS analysis of time-dependant uptake by GM-CSF differentiated BMDC incubated with the benzyl-loaded HSA-nanocarrier or HSA on ice at concentrations of 1 nM and 10 nM, n=1. Results show extremely fast association/uptake.
- **Figure 17**: shows T cell proliferation (cpm) of OVA peptide-specific CD8⁺ (OT-I) **(A-C)** or CD4⁺ (OT-II) **(D-F)** after co-culture with GM-CSF-differentiated BMDC which were preincubated with OVA and subsequently with different HSA nanocarrier systems at a concentration of either 1 (A,D), 10 (B,E) or 50 (C,F) nM, n=6.
- **Figure 18**: shows FACS analysis of uptake of different HSA nanocarrier systems by cDC1 - FLT3L differentiated BMDC and immunostimulation after 3 h and overnight incubation time. A), D) Cy5 MFI and MFI fold of CTRL; B), E) CD86 MFI and MFI fold of CTRL; C), F) MHCII MFI and MFI fold of CTRL. Cells were incubated with the different HSA nanocarrier systems at 50 nM, n=4.

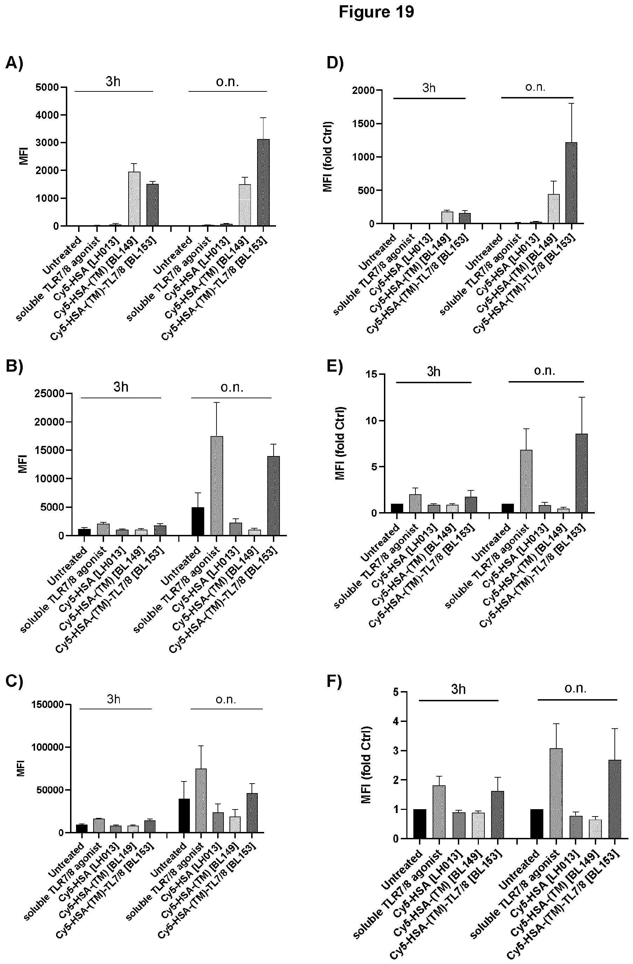

- **Figure 19**: shows FACS analysis of uptake of different HSA nanocarrier systems by cDC2 - FLT3L differentiated BMDC and immunostimulation after 3 h and overnight incubation time. A), D) Cy5 MFI and MFI fold of CTRL; B), E) CD86 MFI and MFI fold of CTRL; C), F) MHCII MFI and MFI fold of CTRL. Cells were incubated with the different HSA nanocarrier systems at 50 nM, n=4.

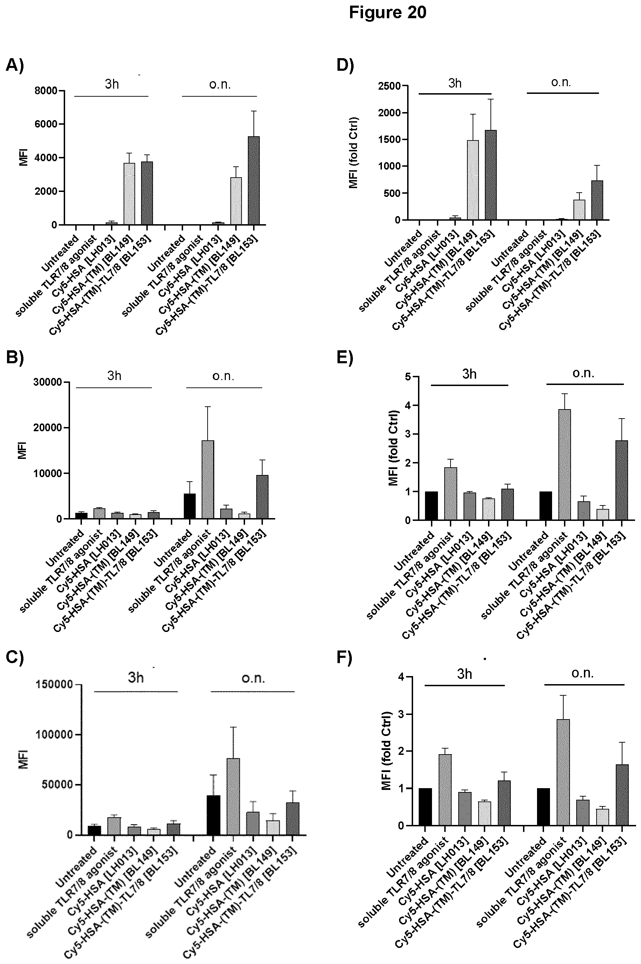

- **Figure 20**: shows FACS analysis of uptake of different HSA nanocarrier systems by pDC - FLT3L differentiated BMDC and immunostimulation after 3 h and overnight incubation time. A), D) Cy5 MFI and MFI fold of CTRL; B), E) CD86 MFI and MFI fold of CTRL; C), F) MHCII MFI and MFI fold of CTRL. Cells were incubated with the different HSA nanocarrier systems at 50 nM, n=4.
- **Figure 21**: shows FACS analysis of uptake of different HSA nanocarrier systems by splenic DC and immunostimulation after 3 h and overnight incubation time. A), D) Cy5 MFI and MFI fold of CTRL; B), E) CD86 MFI and MFI fold of CTRL; C), F) CD209 MFI and MFI fold of CTRL. Cells were incubated with the different HSA nanocarrier systems at 50 nM, n=3.

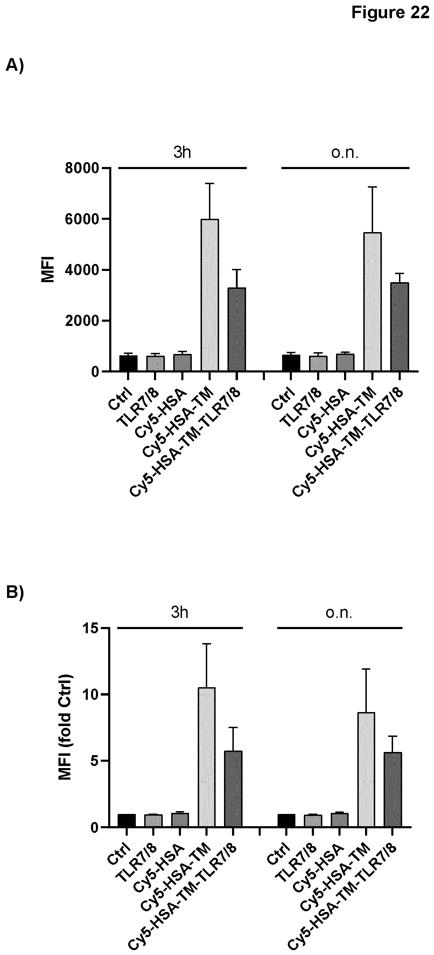

- **Figure 22**: shows FACS analysis of uptake of different HSA nanocarrier systems by CD3⁺ splenic T cells. A), B): Cy5 MFI and MFI fold of CTRL. Cells were incubated with the different HSA nanocarrier systems at 50 nM, n=3.

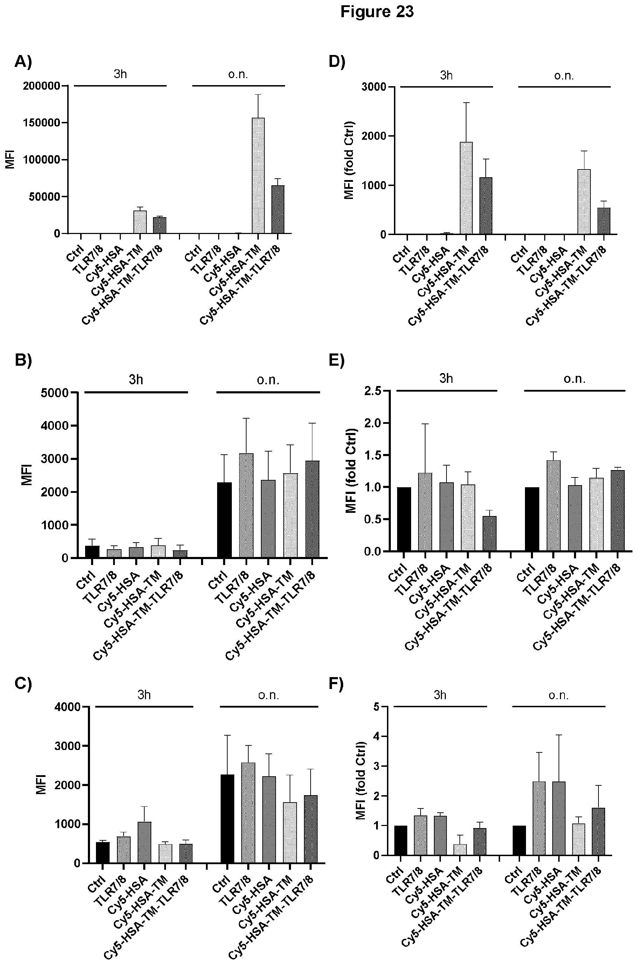

- **Figure 23**: shows FACS analysis of uptake of different HSA nanocarrier systems by splenic macrophages and immunostimulation after 3 h and overnight incubation time. A), D) Cy5 MFI and MFI fold of CTRL; B), E) CD86 MFI and MFI fold of CTRL; C), F) CD209 MFI and MFI fold of CTRL. Cells were incubated with the different HSA nanocarrier systems at 50 nM, n=3.

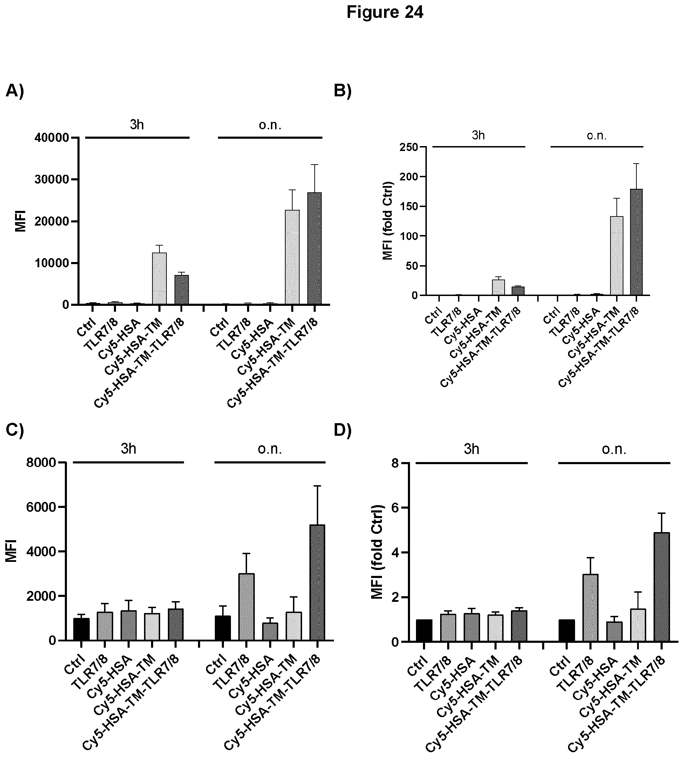

- **Figure 24**: shows FACS analysis of uptake of different HSA nanocarrier systems by splenic PMN and immunostimulation after 3 h and overnight incubation time. A), B) Cy5 MFI and MFI fold of CTRL; C), D) CD86 MFI and MFI fold of CTRL. Cells were incubated with the different HSA nanocarrier systems at 50 nM, n=3-5.

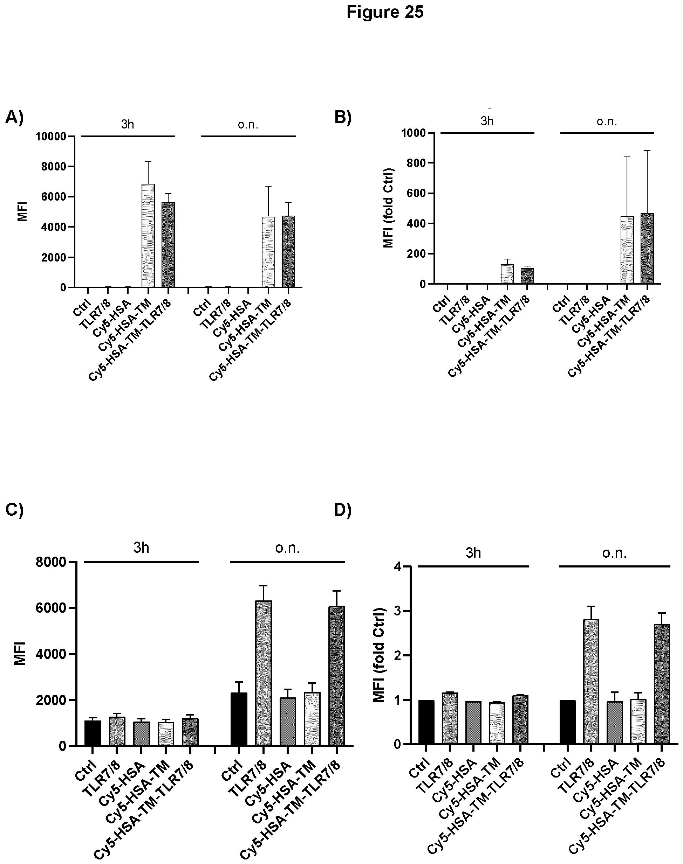

- **Figure 25**: shows FACS analysis of uptake of different HSA nanocarrier systems by splenic B cells and immunostimulation after 3 h and overnight incubation time. A), B) Cy5 MFI and MFI fold of CTRL; C), D) CD86 MFI and MFI fold of CTRL. Cells were incubated with the different HSA nanocarrier systems at 50 nM, n=3.

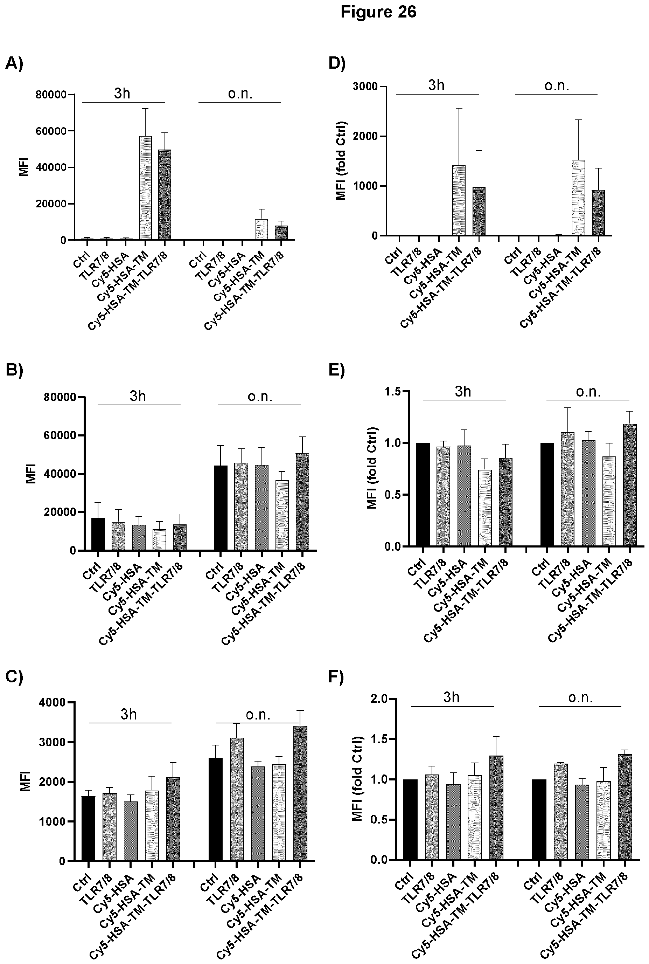

- **Figure 26**: shows FACS analysis of uptake of different HSA nanocarrier systems by liver NPC - CD11c⁺ DC and immunostimulation after 3 h and overnight incubation time. A), D) Cy5 MFI and MFI fold of CTRL; B), E) CD86 MFI and MFI fold of CTRL; C), F) CD209 MFI and MFI fold of CTRL. Cells were incubated with the different HSA nanocarrier systems at 50 nM, n=3-4.

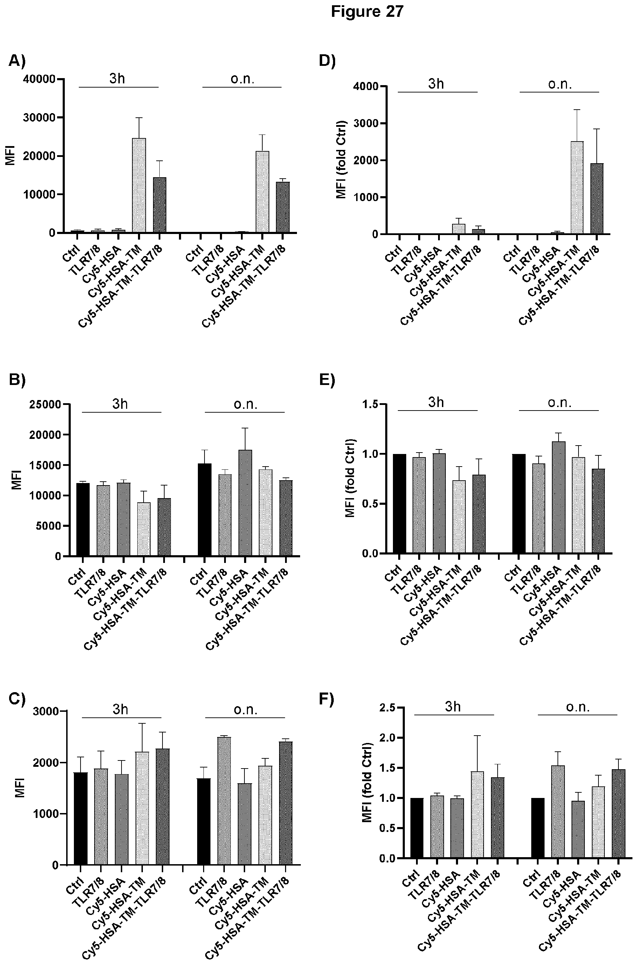

- **Figure 27**: shows FACS analysis of uptake of different HSA nanocarrier systems by iver NPC - F480⁺ Kupffer cells. A), D) Cy5 MFI and MFI fold of CTRL; B), E) CD86 MFI and MFI fold of CTRL; C), F) CD209 MFI and MFI fold of CTRL. Cells were incubated with the different HSA nanocarrier systems at 50 nM, n=3-4.

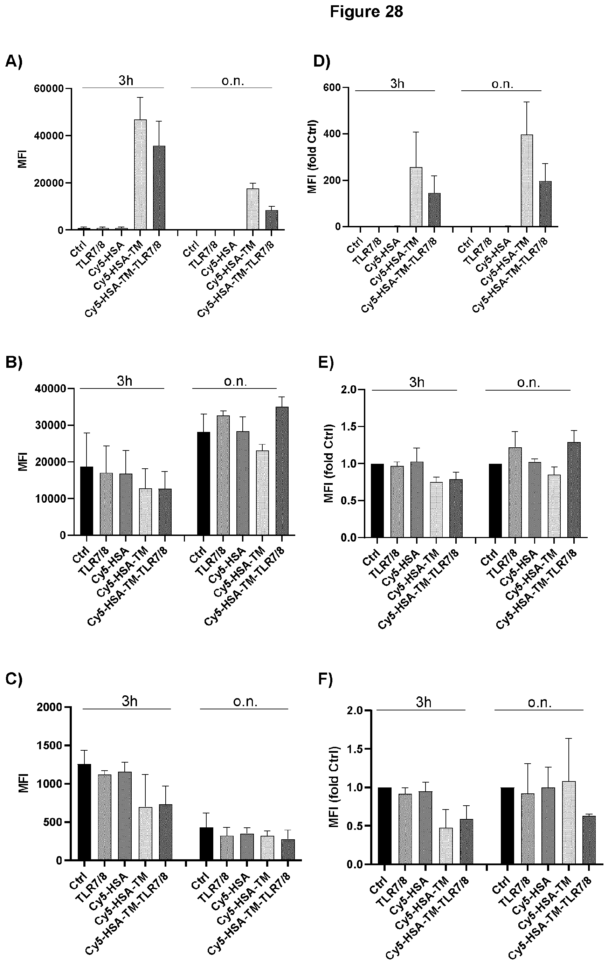

- **Figure 28**: shows FACS analysis of uptake of different HSA nanocarrier systems by liver NPC - CD32b⁺ LSEC immunostimulation after 3 h and overnight incubation time. A), D) Cy5 MFI and MFI fold of CTRL; B), E) CD86 MFI and MFI fold of CTRL; C), F) CD209 MFI and MFI fold of CTRL. Cells were incubated with the different HSA nanocarrier systems at 50 nM, n=3-4.
- **Figure 29**: shows qPCR analysis of phenotype-repolarization of M2-type BMDM. The results show specific regulation of markers of M1- and M2-polarized BMDM after treatment with the indicated HSA nanocarrier systems or the drug CuB alone at a concentration of 250 nM ("Low") or 1 µM ("High") related to the drug. RNA fold change is calculated compared to M2-polarized untreated macrophage control. A) CD206; B) Arg-1; C) YM1; D) iNOS; E) TNFa. CD206, Arg-1 and YM1 are M2 markers, which decrease upon nanocarrier treatment. iNOS and TNFa are M1 markers, which increase upon nanocarrier treatment but only when the carrier is applied at the higher concentration.
- **Figure** 30:: shows FACS analysis of uptake of different HSA nanocarrier systems by B) GM-CSF- or C)-E) FLT3L- differentiated BMDC and A) immunostimulation by GM-CSF-differentiated BMDC. n=4. A) MFI CD86; B)-E) MFI Cy5. ^{Cy5}HSA-TM-TLR7/8_ag was tested after preincubation with native and heat inactivated mouse serum for 30 min at 37 °C to get indications about protein corona formation which could impede uptake. Cells were incubated with the HSA nanocarrier at 50 nM, n=4.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the scope of the invention as described in the following claims.

### EXAMPLES

### Abbreviations

- TLC: thin-layer chromatography
- BMDC: bone marrow derived dendritic cell
- BMDM: bone marrow-derived macrophage
- CHO: chinese hamster ovary cell
- CuB: Cucurbitacin B
- DC: dendritic cell
- DLS: dynamic Light Scattering
- FCS: fluorescence correlation spectroscopy
- FLT3L: FMS-like tyrosine kinase 3 ligand
- GM-CSF: granulocyte macrophage colony-stimulating factor
- HPLC-MS: high-performance Liquid chromatography-mass spectrometry
- HSA: human serum albumin
- LSEC: liver sinusoidal endothelial cell
- Mal: maleimide
- NPC: hepatic Non-Parenchymal Cells
- PMN: polymorphonuclear neutrophil
- Pyr-S-S: pyridinyldithio-ethoxy-carbonyl-group
- TM: trimannose

### Materials (synthesis)

MilliQ water (MQ): ultrapure (Type 1) water (resistivity of 18.2 MΩ.cm at 25°C and a TOC value below 5 ppb). Unless otherwise stated, all chemicals were purchased from Sigma Aldrich Merck, VWR, TCI, Acros Organics, AlfaAesar, Arctomsci or Fisher Scientific and used without further purification.

CHO cells were obtained from Prof. Lutz Nuhn. These cells were transfected as described in Martinez-Pomares et al., 2003, Journal of Leukocyte Biology (73), 604-613.

### Example 1

### Trimannose synthesis procedure

**1,2,3,4,6-Penta-*O*-acetyl-α,β-D-mannopyranose S1:** The reaction was conducted using an oven dried Schlenk flask equipped with a magnetic stirring bar. I₂ (0.04 eq.) was dissolved in Ac₂O (1.11 M in substrate) under Argon-atmosphere. The resulting black solution was cooled to a temperature of 0 °C using an ice bath. D-Mannose (1.00 eq.) was added portion by portion. After removing the ice bath, the reaction mixture was stirred at room temperature until reaction control via TLC and HPLC-MS indicated complete consumption of the starting material and product formation. The reaction mixture was diluted with the same volume of DCM, then washed twice with the same volume of an ice cold saturated aqueous solution of Na₂S₂O₃ and four times with the same volume of a saturated aqueous solution of NaHCOs. The separated organic layer was dried over anhydrous Na₂SO₄, filtered and all volatiles were removed in vacuo to afford the desired product **S1** with quantitative yield as mixture of anomers (α:β 4:1) in form of a colorless solid.

**Phenyl 2,3,4,6-Tetra-*O*-acetyl-1-thio-α-D-mannopyranoside S2:** The reaction was conducted using an oven dried Schlenk flask equipped with a magnetic stirring bar. **S1** (1.00 eq.) was dissolved in dry DCM (0.7 M in substrate) under Argon-atmosphere and molecular sieves 3Å (1/10 of substrate weight) were added. Thiophenol (1.60 eq.) was added using a syringe. The brown mixture was stirred at room temperature (30 minutes at 10 g scale) and then cooled to a temperature of 0 °C using an ice bath. BF₃·OEt₂ (5.00 eq.) was added dropwise using a syringe. The resulting yellow reaction mixture was stirred, while slowly warming up to room temperature, showing a color change to deep red until reaction control via TLC and HPLC-MS indicated complete consumption of the starting material and product formation. The mixture was diluted with the same volume of DCM and poured into twice the volume of ice cooled H₂O. The separated organic phase was washed with the same volume of a saturated aqueous solution of NaHCOs, separated again and poured into the double volume of an ice cooled solution of NaOH with vigorous stirring. After stirring at 0 °C (60 minutes at 10 g scale) the organic phase was separated and washed again with the same volumes of a saturated aqueous solution of NaHCOs, a 1 M solution of NaOH and finally a saturated solution of NaCl. The separated organic layer was dried over anhydrous Na₂SO₄, filtered and all volatiles were removed in vacuo to afford the crude product in form of an orange oil. Purification using FCC (*^{c}*Hex/EtOAc, isocratic with 33 % EtOAc) delivered the desired product **S2** with a yield of 86 % in form of a colorless solid.

**Propargyl α-D-mannopyranoside S3:** Both reaction steps were conducted using oven dried Schlenk flasks equipped with magnetic stirring bars. **S1** (1.00 eq.) was dissolved in dry DCM (0.2 M in substrate) under Argon-atmosphere. The colorless solution was cooled to a temperature of 0 °C using an ice bath. Propargyl alcohol (5.00 eq.) and BF₃·OEt₂ (10.0 eq.) were added dropwise one after another. The resulting yellow reaction mixture was stirred, while slowly warming up to room temperature, showing a color change to deep red until reaction control via TLC and HPLC-MS indicated complete consumption of the starting material and product formation. The reaction mixture was diluted with the same volume of DCM, washed three times with the same volume of H₂O, three times with the same volume of a saturated aqueous solution of NaHCOs and once with the same volume of a saturated solution of NaCl. The separated organic layer was dried over anhydrous Na₂SO₄, filtered and all volatiles removed in vacuo. The crude intermediate product was obtained in form of a yellow oil and suspended in dry MeOH (0.4 M) under Argon-atmosphere. NaOMe (0.10 eq.) was added. The reaction mixture was stirred at room temperature with slow dissolution of the substrate until reaction control via TLC and HPLC-MS indicated complete consumption of the starting material and product formation. Subsequently, the solution was neutralized using Amberlite IR120 H⁺ ion exchange resin until a neutral solution was obtained. The mixture was filtered over *Celite,* which was washed thoroughly with MeOH. All volatiles were removed in vacuo to afford the crude product in form of a yellow foam, which was dissolved in H₂O (0.1 M), washed twice with the same volume of a mixture of *^{c}*Hex and EtOAc (10:1 *v*:*v*)*.* The desired product **S3** was obtained with a yield of 98 % after lyophilization of the aqueous phase and co-distillation with MeOH in form of a colorless solid.

**Propargyl 2,4-Di-*O*-benzoyl-α-D-mannopyranoside S4:** The first two reaction steps were conducted using oven dried Schlenk flasks equipped with magnetic stirring bars. **S3** (1.00 eq.) was dissolved in dry DMF (0.5 M in substrate) under Argon-atmosphere and the solution cooled to a temperature of 0 °C using an ice bath. One after another TBDMSCI (3.00 eq) and imidazole (6.00 eq.) were added. The yellow reaction mixture was stirred at a temperature of 0 °C until reaction control via TLC and HPLC-MS indicated complete consumption of the starting material and product formation. The mixture was diluted with the fivefold amount of DCM and washed once with the same volume of a saturated solution of NaHCOs, twice with the same volume of H₂O and once with the same volume of a saturated solution of NaCl. The collected aqueous phases were extracted with half the volume of DCM. The collected organic phases were dried over anhydrous Na₂SO₄, filtered and all volatiles were removed in vacuo. The first crude intermediate product was obtained in form of a colorless oil and dissolved in dry pyridine (0.7 M) under Argon-atmosphere. Molecular sieves 3 Å (1/10 of substrate weight) were added and the mixture stirred at room temperature (15 min at 5 g scale) before DMAP (0.20 Äq.) was added. Subsequently a solution of benzoyl chloride (5.50 eq.) in dry pyridine (3.4 M in reagent), which was also stirred with molecular sieves 3 Å (1/10 of reagent weight) at room temperature before (15 min at 5 g substrate scale), was added slowly using a syringe. At room temperature. The colorless reaction mixture was stirred at a temperature of 50 °C under Argon-atmosphere showing color change to orange and formation of a colorless precipitation until reaction control via TLC and HPLC-MS indicated complete consumption of the starting material. After cooling to room temperature, the mixture was diluted with the fivefold amount of EtOAc and washed three times with half the volume of H₂O. The collected aqueous phases were extracted once with one third of their volume of EtOAc. The collected organic phases were dried over anhydrous Na₂SO₄, filtered and all volatiles were removed in vacuo. The second crude intermediate product was obtained as a colorless oil and dissolved in THF (0.3 M) and AcOH (1/20 of THF volume) was added. The solution was cooled to a temperature of 0°C using an ice bath. Subsequently a 30 % solution of hydrogen fluoride pyridine complex in pyridine (1/5 of THF volume) was added dropwise at this temperature. After complete addition (45 minutes at 5 g scale) the reaction mixture was stirred while slowly warming up to room temperature until reaction control via TLC and HPLC-MS indicated complete consumption of the starting material. The mixture was diluted with the same volume of DCM and washed three times with half the volume of NaHCOs. The collected aqueous phases were extracted once with one third of their volume of DCM. The collected organic phases were dried over anhydrous Na₂SO₄, filtered and all volatiles were removed in vacuo. The final crude product was obtained in form of a yellow oil. Purification using FCC (*^{c}*Hex/EtOAc, gradient 0 % to 5 % to 100 % EtOAc) as well as RP-FCC (MeCN/H₂O, gradient 10 % to 100 % MeCN) delivered the desired product S4 with a yield of 56 % in form of a colorless foam.

**Propargyl 2,4-Di-*O*-benzoyl-3,6-di-*O*-(2,3,4,6-tetra-*O*-acetyl-α-D-mannopyranosyl)-α-D-mannopyranoside S5:** The reaction was conducted using an oven dried Schlenk flask equipped with a magnetic stirring bar. **S4** (1.00 eq.) and **S2** (2.6 eq.) were co-evaporated for three times with toluene (0.05 M in **S4**) and then dissolved in dry DCM (0.08 M in **S4**) under Argon-atmosphere. Molecular sieves 3Å (1/10 of substrate weight) were added and the solution stirred at room temperature (30 minutes in 0.5 g scale). Subsequently the reaction mixture was cooled to a temperature of -40°C using a cryostat. Silver triflate (0.45 eq.) dissolved in dry toluene (0.22 M in reagent) was added followed by the addition of *N*-iodosuccinimide (4.50 eq.). The solution was slowly warmed up to a temperature of -20°C and stirred at this temperature (4 hours at 0.5 g scale). The reaction mixture was stirred until reaction control via TLC and HPLC-MS indicated complete consumption of **S4** and product formation. The mixture was diluted with the double volume of DCM and washed with three times of the fivefold volume of a saturated solution of NaHCOs. The collected aqueous phases were extracted once with one third of their volume of DCM. The collected organic phases were dried over anhydrous Na₂SO₄, filtered and all volatiles were removed in vacuo. The crude product was obtained in form of a brown oil. Purification using FCC (*^{c}*Hex/EtOAc, gradient 0% to 5 % to 100% EtOAc) as well as RP-FCC (MeCN/H₂O, gradient 10 % to 100 % MeCN) delivered the desired product **S5** with a yield of 60 % in form of a colorless foam.

**1,11-Di[(p-toluolsulfonyl)oxy]-3,6,9-trioxaundecane S6:** The reaction was conducted using an oven dried Schlenk flask equipped with a magnetic stirring bar. Tetraethylenglycol (1.00 eq.) was stirred in high vacuum (30 minutes for 20 g scale) and subsequently dissolved in dry DCM (1 M in substrate) under Argon-atmosphere. The mixture was cooled to a temperature of 0 °C using an ice bath. Then *p*-TsCl (2.40 Äq.) was added and the reaction mixture stirred while warming to room temperature until the reagent was completely dissolved. The mixture was again cooled to a temperature of 0 °C using an ice bath and pulverized KOH (7.80 eq.) was added portion wise so that only mild boiling occurred. Upon complete addition the resulting cloudy solution was stirred while slowly warming up to room temperature with formation of a colorless precipitation occurring. The reaction mixture was stirred until reaction control via TLC and HPLC-MS indicated complete consumption of the substrate and product formation. The reaction mixture was diluted with the same volume of DCM and then twice the volume of ice cooled H₂O was added. The separated aqueous phase was extracted three times with half the volume of DCM. The collected organic phases were washed with the same volume of H₂O and the same volume of a saturated solution of NaCl, dried over anhydrous Na₂SO₄, filtered and all volatiles were removed in vacuo. The crude product was obtained in form of a yellow oil. Purification using FCC (*^{c}*Hex/EtOAc, gradient 0 % to 40 % to 100 % EtOAc) delivered the desired product **S6** with a yield of 96 % in form of a colorless oil.

**1,11-Diazido-3,6,9-trioxaundecane S7:** The reaction was conducted using an oven dried Schlenk flask equipped with a magnetic stirring bar. **S6** (1.00 eq.) was dissolved in dry DMF (0.5 M in substrate) under Argon-atmosphere.

NaNs (2.50 eq.) and TBAI (0.05 eq.) were added one after the other. The yellow reaction mixture was stirred at a temperature of 80 °C with formation of a colorless precipitate occurring until reaction control via TLC and HPLC-MS indicated complete consumption of the substrate and product formation. The mixture was filtered over *Celite* and thoroughly eluted with Et₂O. All volatile components were removed in vacuum and then high vacuum. The obtained residue was co-evaporated for three times with toluene (1/4 volume of DMF), then dissolved in Et₂O (1/4 volume of DMF) and again filtered over *Celite,* to remove undissolved salts. All volatile components were removed in vacuo and the desired product **S7** was obtained with a yield of 97 % in form of a yellow oil.

**(1-(11-Azido-3,6,9-trioxaundec-1-yl)-1*H*-1,2,3-triazol-4-yl)-methyl 2,4-Di-*O-*benzoyl-3,6-di-*O*-(2,3,4,6-tetra-*O*-acetyl-α-D-mannopyranosyl)-α-D-manno-pyranoside S8:** The reaction was conducted using an oven dried Schlenk flask equipped with a magnetic stirring bar. **S5** (1.00 eq.) and **S7** (15.0 eq.) were dissolved in dry DMF (0.01 M in substrate), which was degassed using ultrasonication while introducing a stream of Argon gas. The solution again was degassed by conducting three freeze-pump-thaw-cycles. Subsequently PMDTA (0.85 eq.) was added, and the mixture warmed to a temperature of 45 °C followed by the addition of Cu(I)Br (0.50 eq.). The resulting turquoise reaction mixture was stirred at a temperature of 45 °C until reaction control via TLC and HPLC-MS indicated complete consumption of **S5** and product formation. The reaction mixture was allowed to cool to room temperature and diluted with twice the volume of EtOAc. The organic layer was washed twice with the same volume of a saturated aqueous solution of NH₄Cl. The aqueous phase was diluted with H₂O to dissolve the precipitated salts and extracted with the same volume of EtOAc. The combined organic layers were dried over anhydrous Na₂SO₄, filtered and all volatile components were removed in high vacuum. The crude product was obtained in form of a yellow liquid. Purification using FCC (*^{c}*Hex/EtOAc, gradient 0 % to 100 % EtOAc) delivered the desired product **S8** with a yield of 97 % in form of a colorless oil. Additional **S7** (12.8 eq.) were recovered corresponding to a recovery rate of 92 %.

**(1-(11-Azido-3,6,9-trioxaundec-1-yl)-1*H*-1,2,3-triazol-4-yl)-methyl 3,6-Di-*O*-(α-D-mannopyranosyl)-α-D-mannopyranoside TM:** The reaction was conducted using an oven dried Schlenk flask equipped with a magnetic stirring bar. **S8** (1.00 eq.) was dissolved in dry MeOH (0.05 M in substrate) under Argon-atmosphere. NaOMe (1.00 eq.) and NaOH (0.10 eq.) were added, and the reaction mixture was stirred at a temperature of 35 °C with slow dissolution of the substrate until reaction control via TLC and HPLC-MS showed complete conversion of the substrate and product formation. The solution was neutralized using Amberlite IR120 H⁺ ion exchange resin until a neutral solution was obtained. The mixture was filtered over *Celite* and was thoroughly eluted with MeOH. All volatiles were removed in vacuo. The obtained residue was dissolved in H₂O and washed three times with the same volume of Et₂O. After lyophilization of the aqueous phase, the crude product was obtained in form of a light-yellow oil. Purification using RP-FCC (MeCN/H₂O, gradient 10 % to 100 % MeCN) delivered the desired product **TM** with quantitative yield in form of a colorless lyophilizate.

### Optimal synthesis of loaded trimannose-HSA-Carrier (Figure 3, 5)

In a first **"step i.",** HSA (Sigma Aldrich, ≥96% purity) is optionally labeled with Cyanine5 through reaction of a cysteine thiol residue of HSA with Sulfo-Cyanin5-Maleimid (Mal-SCy5, Lumiprobe) to obtain ^{Cy5}HSA (Fig. 5B). To this aim, HSA (1 eq) is dissolved in 50mM sodium PB pH 7.4 to obtain a protein concentration of 5 mg/mL. Sulfo-Cy5 maleimide (1.5 eq) dissolved in MQ (25 mg/mL) is added to the HSA solution. The reaction is shaken at room temperature for 4-6 hours and afterwards purified (5 × MQ) and concentrated via ultracentrifugation spinfilter (Vivaspin, PES, 10 kDa). The product is lyophilized and obtained as blue powder. Recovery Rate: 94%.

Thereafter, the following procedure is performed for maleimide "Ring Opening": ^{Cy5}HSA was diluted in 50mM sodium borate buffer pH 9.2 to a final protein concentration of 0.5 mg/mL and shaken at 37 °C for 24 h. Afterwards, it was purified (5 × MQ) and concentrated via ultracentrifugation spinfilter (Vivaspin, PES, 10 kDa). The product was lyophilized and obtained as blue powder. Recovery Rate: 99%, DoL: 0.66. This reaction to open the maleimide ring is irreversible and allows for higher stability.

In **"step ii.",** lysine residues of native HSA are modified using NHS-chemistry to attach the linker dibenzoazacyclooctyne (DBCO)-groups to the protein surface (Conju-Probe CP-2028 and Jena Bioscience CLK-A134-10), and obtain **^{Cy5}HSA-(DBCO)₄₂ (****Fig. 5C****).** To this aim ^{Cy5}HSA (1 eq) was diluted in 50mM PB pH 7.4 to a protein concentration of 1 mg/mL. DBCO-PEG₄-NHS (200 eq) dissolved in DMSO (100 mg/mL) was added in portions. Degree of Lysine modification was monitored with Maldi-ToF-MS. Afterwards, the reaction solution was purified (5xMQ) and concentrated via ultracentrifugation spinfilter (Vivaspin, PES, 10 kDa). The product was lyophilized and obtained as blue powder. Recovery Rate: 93%.

The glycolytic targeting structure, trimannose (TM), was synthesized with an azide as reactive handle, to click by a "strain-promoted copper free azide alkyne click" to the DBCO-groups and obtain **^{Cy5}HSA-(TM)₃₄ (Step iii.,** **Fig. 3****,** **Fig. 5D****).** To this aim ^{Cy5}HSA-(DBCO)₄₂ (1 eq) was dissolved in 50 mM PB with 8 M Urea and 2 mM EDTA pH 8 to a final protein concentration of 1 mg/mL. Trimannose-azide (2 eq/DBCO) was dissolved in MQ (10 mg/mL) and added and the reaction solution was shaken at RT overnight. Afterwards, the reaction solution was purified (5 × MQ) and concentrated via ultracentrifugation spinfilter (Vivaspin, PES, 10 kDa). The product was lyophilized and obtained as blue powder. Recovery Rate: 97%.

In "**step iv.",** the trimannosylated HSA is then completely denatured including the reduction of the native disulfides. Denaturation was accomplished by dissolving ^{Cy5}HSA-(TM)₃₄ (1 eq) in denaturing buffer (50 mM PB + 8 M Urea + 2 mM EDTA) and inverting for 20 min before TCEP (150 eq) dissolved in reaction buffer (40mM) was added. The reaction solution was inverted for 1 h and the residual TCEP was removed via ultracentrifugation spinfilter (2 × denaturing buffer, Vivaspin, PES, 10 kDa).

In **"step v**.", the cargo is loaded via a disulfide exchange reaction. The free thiols of HSA resulting from the denaturation step react with a thiol-reactive functional group attached to one drug ("cargo") of choice. The added thiol-reactive functional groups were a pyridinyldithio-ethoxy-carbonyl-group ("Pyr-S-S") or a maleimide ("Mal").

For that, "Pyr-S-S-TLR7/8", or "Pyr-S-S-CuB", and "Pyr-S-S-Benzyl" were obtained as shown in **Figure 2****.** Compound **3** was obtained by the following procedure: In a dry round bottom flask 2,2'-dipyridyldisulfide **1** (3 eq) was dissolved in dry EtOH (1 M) under argon atmosphere. 2-Mercaptoethanol was added dropwise to the reaction solution and the reaction solution was stirred for 2 h at room temperature. The solvent was removed under reduced pressure and the product was purified via column chromatography (EA:hexane; 8:2). The product, 2-(2-pyridinyldithio)ethanol **2,** was obtained as colorless solid (985%). 2-(2-Pyridinyldithio)ethanol **2** (1 eq) and NEts (3 eq) were dissolved in DCM (0.26 M), cooled to 0 °C and 4-nitrophenyl chloroformate (1.1 eq) were added to the reaction. The reaction was allowed to warm to room temperature and stirred for 4 h at room temperature. The reaction mixture was washed twice with water, dried over MgSO₄ and the solvent was removed under reduced pressure. The product, 4-nitrophenyl 2-(2-pyridinyldithio)ethyl carbonate **3,** was purified via column chromatography (Hexane:EA; 1:1) and was obtained as yellow oil (82%). Drugs were modified with compound **3** to form carbonates/carbamates:
4-nitrophenyl 2-(2-pyridinyldithio)ethyl carbonate **3** (1 eq) was dissolved in dichloromethane (68 mM) and added to CuB (1 eq) 4-(dimethylamino)pyridin (1 eq) in dichloromethane (168 mM) was added. The reaction was shaken overnight at room temperature. Dichloromethane was evaporated and the crude mixture was purified via RP-HPLC. After lyophilization the product was obtained as colorless solid (47% as TFA salt).
4-nitrophenyl (2-(pyridin-2-yldisulfaneyl)ethyl) carbonate (1 eq) was dissolved in dichloromethane (2.8 M) and mixed with benzylamine (1 eq). The reaction was shaken overnight at room temperature. Dichloromethane was evaporated and the crude mixture was purified via RP-HPLC. After lyophilization the product was obtained as colorless solid (59% as TFA salt).

The TFA salt of 1-(4-(Aminomethyl)benzyl)-2-butyl-1H-imidazo[4,5-c]quinolin-4-amine (TLR7/8-Agonist) (1 eq) was dissolved in DMF (76 mM) and diisopropylethylamine (DIPEA) (1.3 eq) was added. Subsequently, 4-nitrophenyl (2-(pyridin-2-yldisulfaneyl)ethyl) carbonate (1eq) dissolved in dichloromethane (2.8 M) was added. The reaction was shaken overnight at room temperature. Dichloromethane was evaporated and the crude mixture was purified via RP-HPLC. After lyophilization the product was obtained as colorless solid (72% as TFA salt).

The covalent loading of Pyr-S-S-drug was performed via disulfide exchange reaction with thiols from HSA cysteine residues (HSA-disulfide bridges were cleaved priorly) releasing 2-mercaptopyridin as byproduct.

To the HSA protein solution (1 mg/mL) "Pyr-S-S-TLR7/8" (150 eq), or "Pyr-S-S-CuB" (150 eq), or "Pyr-S-S-Benzyl" (300 eq, control cargo) dissolved in DMF (40mM) was added. The reaction solution was inverted for 1-3 h and the buffer exchanged via ultracentrifugation (3 × denaturing buffer) before N-Ethylmaleimide (150 eq) was added to cap residual free thiols on the HSA for 14 h. The product was purified (5 × denaturing buffer, 5 × MQ) and concentrated via ultracentrifugation spin filter (Vivaspin, PES, 10 kDa). The product was lyophilized and obtained as blue powder. Recovery Rate: 34%. The recovery rate varied depending on cargo and reaction scale: Molecular ratio of drug loading was calculated by Maldi-ToF-MS and varied in the following ranges: TLR7/8:HSA = 20-21; Benzyl:HSA= 12-25. In particular:

| | |
|---|---|
| Batch BL149 ^{Cy5}HSA-(TM)₄₂-Benzyl: | Benzyl:HSA = 25.2 : 1; |
| Batch BL151 ^{Cy5}HSA-(TM)₄₂-CuB: | CuB:HSA = 14.8 : 1 |
| Batch BL153 ^{Cy5}HSA-(TM)₄₂-TLR7/8_ag: | TLR7/8_ag:HSA =20.5 : 1 |
| Batch BL185 ^{Cy7.5}HSA-(TM)₃₈-Benzyl: | Benzyl-HSA = 12 : 1 |
| Batch BL186 ^{Cy7.5}HSA-(TM)₃₈-TLR7/8_ag: | TLR7/8_ag:HSA = 20.8 : 1 |

Unreacted thiols are capped with N-ethylmaleimide (Figure 3, step v., 2. reactant).

Afterwards, all reactive substances are washed out and the protein is folded via rapid dilution procedure, which does not lead to its exact native conformation but to an α-helix-based globular structure (**step vi., "backfolding"**). Single HSA-constructs are obtained with less than 8% of aggregates or multi-HSA constructs according to FCS measurements.

Synthesis of the nanocarrier for *in vivo* experiments (e.g. mouse *in vivo* experiments).

The synthesis procedure to obtain a nanocarrier suitable for "in vivo" experiments differs from the procedure described above for the following aspects (**Figure 4**):
1) the free thiol residue of the cysteine of HSA was capped using N-ethylmaleimide;
2) HSA was labeled with a Near-Infrared Dye (Sulfo Cy7.5-NHS) at a degree of labeling dye:HSA = [2-3] to allow for imaging in the mouse;
3) the dye was attached by NHS-ester chemistry because the maleimide contained in the dye molecule must be ring opened to guarantee *in vivo* stability and the NIR-dyes are chemically sensitive to ring opening conditions.

The following table describes the type of linkages in an exemplary TM-HSA nanocarrier synthesized using a NHS-PEG-DBCO linker and N₃-TM.

**Table 2 Types of linkages in an exemplary TM-HSA nanocarrier system.**

| Component 1 | Reactive chemical function 1 | Component 2 | Reactive chemical function 2 | Product | Linkage / bond type | Replaceable by alternative chemical strategy |
|---|---|---|---|---|---|---|
| HSA | Thiol | Dye | Maleimide | Dye-HSA | Thioether: covalent, reversible | yes |
| HSA | Primary amine | Dye | NHS ester | Dye-HSA | Amide: covalent, irreversible | yes |
| Dye-HSA | Primary amine | NHS-PEG4-DBCO | NHS ester | Dye-DBCO-HSA | Amide: covalent, irreversible | yes |
| Dye-DBCO-HSA | DBCO | Trimannose-azide | Azide | Dye-TM-HSA | Triazol: covalent, irreversible | Yes |
| Dye-TM-HSA | Internal disulfide bridges | Reducing agent TCEP | Phosphine | Dye-TM-HSA-SH (reduced) | Disulfide reduction to thiols: reversible | Reducing agent: yes Reducing step: No |
| Dye-TM-HSA-(SH) | Internal free thiols | Pyr-SS-Cargo | Disulfide bridge | Dye-TM-HSA-Cargo | Disulfide bridge: covalent, reversible | yes |
| Dye-TM-HSA-(SH) | Internal free thiols | Maleimide-Cargo | Maleimide | Dye-TM-HSA-Cargo | Thioether: covalent, reversible | yes |

### Example 2

### Characterization of the drug-loaded trimannose-HSA nanocarrier systems.

Dynamic Light Scattering (DLS) (Figure 8), Matrix Assisted Laser Desorption/Ionization Time of Flight mass spectrometry (Maldi-ToF-MS), Fluorescence Correlation Spectroscopy (FCS) (Figure 6), and circular dichroism spectrometry (CD) Secondary Structure Analysis (Figure 7), agarose gel electrophoresis and SDS page (Figure 9).

**CD** spectra were recorded on a JASCO J-1500 spectrometer in a 0.1 cm High Precision Cell by HellmaAnalytics. The recorded data were processed in Spectra Analysis by JASCO. For the measurement compounds were dissolved in 10 mM sodium phosphate buffer pH 7.4 (800 nM). CD spectra were recorded at wavelengths from 260 to 180 nm with a bandwidth of 1 nm, data pitch of 0.2 nm and a scanning speed at 20 nm min⁻¹ at 37 °C. Spectrum analysis was performed using BeStSell (https://bestsel.elte.hu) for secondary structure determination (analysis parameter: wavelength range 190-250 nm, scale factor 4.9 (HSA) 1.7 (BL153), RMSD 0.2774 (HSA) 0.2807 (BL153), NRMSD 0.01229 (HSA) 0.01283 (BL153). Secondary structure analysis indicates that TLR7/8-agonist loaded nanocarrier shows an α-helix rich structure similar to native HSA.

**MALDI-TOF-MS** spectra were acquired on a Bruker Time-of-flight MS rapifleX (matrix: sinapinic acid). Spectra were analyzed and processed (baseline correction, spectrum smoothening) with mMass software. Maldi-ToF-MS spectra show the molecular weight increase of final nanocarriers compared to native HSA.

**DLS** measurements of the compounds (1mg/mL in PBS or MQ) were performed at 25 °C using a Malvern ZetaSizer Nano S purchased from Malvern Instruments Ltd. (Malvern, Great Britain) with a He/Ne Laser (λ = 633 nm) and a narrow band filter at a fixed scattering angle of 173°. Volume distribution of DLS measurements of nanocarriers in PBS was used to determine their size range.

**FCS** experiments were performed using a commercial confocal microscope (LSM 880, Carl Zeiss, Jena, Germany) equipped with a C-Apochromat 40×/1.2 W (Carl, Zeiss, Jena, Germany) water immersion objective. A HeNe laser (λ = 633 nm) fiber coupled to the LSM 880 was used for the excitation of the Sulfo-Cyanine5 dye. The emission light in the spectral range 655-699 nm (Cy5) was detected using a spectral detection unit (Quasar, Carl Zeiss) that comprises a diffraction grating and a 32 channel GaAsP photomultiplier array operating in the photon counting mode. An eight-well polystyrene-chambered cover slide (Laboratory-Tek, Nalge Nunc International) was used as a sample cell. For the FCS experiments with nanocarrier in water and blood plasma, a droplet of about 50 µL solution (40 nM) was placed directly on the cover slide. The confocal detection volume was placed ~15 µm above the glass coverslip. The recorded experimental FCS autocorrelation curves were fitted with the theoretical model function for an ensemble of either one or two types of freely diffusing fluorescence species. The fits yielded the diffusion coefficients (D) and through the Stokes-Einstein relation the hydrodynamic radii (R_{H}) of the studied species. For calibration of the confocal volume, a dye with known diffusion coefficient, i.e. Alexa 633 in water was used. FCS experiments show only a slight increase in hydrodynamic radius of TM-^{Cy5}HSA-Benzyl after incubation in blood plasma indicating no major protein corona formation around the nanocarrier. FCS measurements showed a size of 24 nm for a TM-^{Cy5}HSA-Benzyl nanocarrier.

For **agarose gel electrophoresis,** 1% agarose gel (0.5 g agarose, 50 mL 1xTAE buffer) was prepared and samples (20 µL: 30.1 pmol + MQ, 10 µL 50% glycerol solution) were loaded. Gel was run at 150 V for 33 min. Gel was imaged using the Cy5 channel and stained with Coomassie before imaging (BioRad ChemiDoc) using the Coomassie Blue channel. Agarose gel confirmes the expected overall negative charge of HSA nanocarriers and shows no aggregate formation.

For **SDS-PAGE** commercially available Mini-PROTEAN Precast Gel (BioRad) was used. Samples (reducing: 10 µL: 7.5 µL (30.1 pmol*) sample, 2.5 µL 4x Laemmli sample buffer + DTT (200mM); non-reducing: 10 µL: 7.5 µL sample, 2.5 µL 4x Laemmli sample buffer) were prepared, heated to 95 °C for 10 min and loaded (*sample amount was decreased to 15.05 pmol for HSA and ^{Cy5}HSA). Gel was run 10-15 min at 80 V and then 120 min at 120 V. Gel was imaged using the Cy5 channel and stained with Coomassie before imaging using the Coomassie Blue channel (BioRad ChemiDoc). SDS-PAGE gel shows gradual weight increase with increasing modification rate.

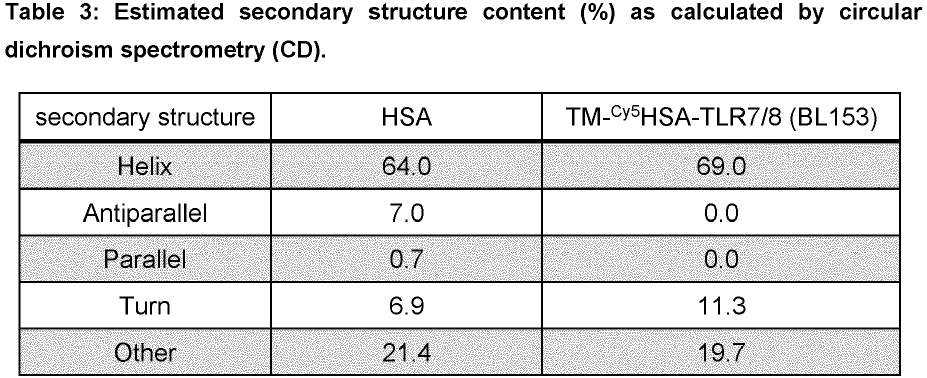

### Example 3

### Analysis of cell uptake by CHO cells expressing surface MMR or not.

CHO^{MMR+} cells were incubated with ^{SCy5}HSA-(TM)_{34.1} 1µM for 24 hours and then analysed at confocal microscopy. Red fluorescence signal showed that only trimannosylated HSA was efficiently uptaken (**Figure 10**).

Experiments of flow cytometry showed that TM-HSA constructs were only uptaken in MMR-positive cells and not in MMR negative cells. Uptake Efficiency correlated positively with amount of TM on HSA surface. HSA without trimannose (Ctrl) was not uptaken in neither cell line (**Figure 11**).

### Example 4 Optimal numbers of TM-groups (Figure 12)

Performed experiments showed no uptake in receptor negative CHO cells indicating specific TM receptor-mediated uptake. Increased TM-Amount on HSA correlates positively with increased uptake. 3-10 TM groups / HSA were sufficient for this transfected cell line. For further studies the inventor still chose >30 TM groups/HSA because a) they stabilize the carrier during denaturation for loading procedure due to hydrophilicity b) they support backfolding after drug loading (hydrophilic parts outside).

### Example 5

### Analysis of cell uptake and immunostimulation of TM-HSA conjugates and DBCO-HSA conjugates by GM-CSF differentiated BMDC, in DCs, LSEC, Kupffer cells. [Figure 13-16]

Graphs show uptake after overnight incubation in various primary cells. "DBCO: 40" (^{Cy5}HSA-(DBCO)₄₀) serves as positive control for unspecific uptake because a) DBCO groups can intercalate into the cell membrane and promote thereby cell internalization and b) so far we can only convert 80% of DBCO groups in the Click reaction with the TM - Therefore, some "free" DBCO groups are always existent, even though it is likely that they are buried within hydrophobic pockets of HSA since they are not reacting with N₃-TM.

Saturation of TM-mediated uptake from around 20 TM/HSA on. Concentration series uptake (top right) confirms saturation of cells since no increased uptake is observed in increased concentrations.

Carrier/TM does not induce DC-SIGN upregulation/expression (bottom right) in GM-CSF DC.

### Example 6

### Analysis of OTI/OTII T cell proliferation after incubation with DC and different HSA-nanocarriers. [Figure 17]

GM-CSF-differentiated BMDC were incubated with OVA. 3 h later, nanocarriers were added at a concentration of either 1 **(A,D),** 10 **(B,E)** or 50 **(C,F)** nM and incubated overnight (o.n.). The next day, serially titrated numbers of harvested and washed DC (starting with 10⁴/100 µL) were co-cultured with immune magnetically sorted OVA peptide-specific CD8⁺ (OT-I) **(A-C)** or CD4⁺ (OT-II) **(D-F)** T cells (5 × 10⁴/100 µL) in triplicate in 96-well plates. Proliferation of T cells was assessed by incorporation of ³H-thymidin applied for the last 16 h of 3-4 days of co-culture. Data denote the mean of two compiled experiments performed in triplicate.

### Example 7

### Analysis of cell uptake and immunostimulation in FLT3L differentiated BMDC. [Figure 18-20]

FLT3L-differentiated BMDC were incubated with nanocarriers for 3 h or overnight (o.n.), respectively, stained and subjected to flow cytometric analyses. cDC1 subpopulation was defined as follows: FVD⁻CD11c⁺ XCR1⁺, n=4 (Figure 18). cDC2 subpopulation was defined as follows: FVD⁻CD11c⁺ Sirp1α⁺, n=4 (Figure 19). pDC subpopulation was defined as follows: FVD⁻ CD11c⁺ B220⁺, n=4 (Figure 20). **(A)** MFI or **(D)** to control normalized MFI of Cy5 signal (nanocarrier binding or uptake). **(B)** MFI or **(E)** to control normalized MFI of surface activation marker CD86 expression. **(C)** MFI or **(F)** to control normalized MFI of surface activation marker MHCII expression.

### Example 8

### Analysis of cell uptake and immunostimulation in splenic DC, T cells, B cells, PMN and macrophages. [Figure 21-25]

Spleen cells were incubated with nanocarriers for 3 h or overnight (o.n.), respectively, stained and subjected to flow cytometric analyses. Splenic DC population was discriminated as follows: (fixable viability dye) FVD⁻ CD19⁻ NK1.1⁻CD3⁻ CD11c⁺, n=3 (**Figure 21**)**. (A)** MFI or **(D)** to control normalized MFI of Cy5 signal (nanocarrier binding or uptake). **(B)** MFI or **(E)** to control normalized MFI of surface activation marker CD86 expression. **(C)** MFI or **(F)** to control normalized MFI of DC-SIGN receptor CD209a expression.

Spleen cells were incubated with nanocarriers for 3 h or overnight (o.n.), respectively, stained and subjected to flow cytometric analyses. Splenic T cell population was discriminated as follows: FVD⁻ CD19⁻ NK1.1⁻ CD3⁺, n=3 (**Figure 22**)**. (A)** MFI or **(B)** to control normalized MFI of Cy5 signal (nanocarrier binding or uptake).

Spleen cells were incubated with nanocarriers for 3 h or overnight (o.n.), respectively, stained and subjected to flow cytometric analyses. Splenic macrophage population was discriminated as follows: FVD⁻ CD19⁻ NK1.1⁻ CD3⁻CD11c⁻ CD11b⁺ Ly6G⁻, n=3 (**Figure 23**). **(A)** MFI or **(D)** to control normalized MFI of Cy5 signal (nanocarrier binding or uptake). **(B)** MFI or **(E)** to control normalized MFI of surface activation marker CD86 expression. **(C)** MFI or **(F)** to control normalized MFI of DC-SIGN receptor CD209a expression.

Spleen cells were incubated with nanocarriers for 3 h or overnight (o.n.), respectively, stained and subjected to flow cytometric analyses. Splenic PMN population was discriminated as follows: FVD⁻ CD19⁻ NK1.1⁻ CD3⁻ CD11c⁻ CD11b⁺ Ly6G⁺, n=3. **(A)** MFI or **(B)** to control normalized MFI of Cy5 signal (nanocarrier binding or uptake). **(C)** MFI or **(D)** to control normalized MFI of surface activation marker CD86 expression (**Figure 24****)**.

Spleen cells were incubated with nanocarriers for 3 h or overnight (o.n.), respectively, stained and subjected to flow cytometric analyses. Splenic B cell population was discriminated as follows: FVD⁻ CD19⁺, n=3. **(A)** MFI or **(B)** to control normalized MFI of Cy5 signal (nanocarrier binding or uptake). **(C)** MFI or **(D)** to control normalized MFI of surface activation marker CD86 expression **(****Figure 25****).**

### Example 9

### Analysis of cell uptake and immunostimulation in liver NPC.

Liver NPC were incubated with nanocarriers for 3 h or overnight (o.n.), respectively, stained and subjected to flow cytometric analyses. Liver DC population was discriminated as follows: FVD⁻ CD45⁺ CD11c⁺, n=4 (**Figure 26****). (A)** MFI or **(D)** to control normalized MFI of Cy5 signal (nanocarrier binding or uptake). **(B)** MFI or **(E)** to control normalized MFI of surface activation marker CD86 expression. **(C)** MFI or **(F)** to control normalized MFI of DC-SIGN receptor CD209a expression.

Liver NPC were incubated with nanocarriers for 3 h or overnight (o.n.), respectively, stained and subjected to flow cytometric analyses (**Figure 27****).** Kupffer cell population was discriminated as follows: FVD⁻ CD45⁺ F4/80⁺, n=4. **(A)** MFI or **(D)** to control normalized MFI of Cy5 signal (nanocarrier binding or uptake). **(B)** MFI or **(E)** to control normalized MFI of surface activation marker CD86 expression. **(C)** MFI or **(F)** to control normalized MFI of DC-SIGN receptor CD209a expression.

Liver NPC were incubated with nanocarriers for 3 h or overnight (o.n.), respectively, stained and subjected to flow cytometric analyses (**Figure 28****).** LSEC population was discriminated as follows: FVD⁻ CD45⁺ CD32b⁺, n=4. **(A)** MFI or **(D)** to control normalized MFI of Cy5 signal (nanocarrier binding or uptake). **(B)** MFI or **(E)** to control normalized MFI of surface activation marker CD86 expression. **(C)** MFI or **(F)** to control normalized MFI of DC-SIGN receptor CD209a expression.

### Example 10

### Phenotype-Repolarization of M2-type BMDM , [Figure 29]

M2-polarized BMDM cells were treated with CuB High (1 µM) and CuB Low (250 nM), TM-^{Cy5}HSA-CuB High (1 µM*) and Low (250 nM*) as well as TM-^{Cy5}HSA and ^{Cy5}HSA only as a drug (n=3). Total RNA was isolated and subjected to qPCR analyses. *related to the drug concentration

### Example 11

### Surface activation marker expression and uptake by in vitro differentiated BMDC of nanocarriers after incubation with mouse serum.

**(A,B)** GM-CSF- or **(C-E)** FLT3L-differentiated BMDC were incubated with nanocarriers or R848 (1 µg/ml) o.n., stained and subjected to flow cytometric analyses. GM-CSF differentiated BMDC were discriminated as follows: FVD⁻CD11c⁺. FLT3L-differentiated cells were discriminated as described in Fig. 18-20. n=4. **(A)** MFI of surface activation marker CD86 expression. **(B-E)** MFI of Cy5 signal (nanocarrier binding or uptake). Nanocarriers were pre-incubated for 30 min at 37°C with heat inactivated or native mouse serum.

**Table 4: List of performed uptake experiments**

| **Cells** | **HSA structure** | **Figure** |
|---|---|---|
| **CHO & CHO^{MMR+}** | ^{Cy5}HSA-TM₃₄[KG156]; ^{Cy5}HSA-DBCO₄₀ [KG212]; | **10** |
| **CHO & CHO^{MMR+}** | ^{Cy5}HSA [BL109]; ^{Cy5}HSA-TM₁[KG151]; ^{Cy5}HSA-TM₉[KG153]; ^{Cy5}HSA-TM₂₀[KG154]; ^{Cy5}HSA-TM₃₄[KG156]; ^{Cy5}HSA-DBCO₄₀ [KG212], | **11, 12** |
| **GM-CSF DC** | ^{Cy5}HSA [BL109]; ^{Cy5}HSA-TM₁[KG151]; ^{Cy5}HSA-TM₉[KG153]; ^{Cy5}HSA-TM₂₀[KG154]; ^{Cy5}HSA-TM₃₄[KG158]; ^{Cy5}HSA-DBCO₄₀ [KG212]; | **13, 14** |
| **GM-CSF DC** | ^{Cy5}HSA-TM₄₂[BL149]; | **14** |
| | ^{Cy5}HSA-TM₄₂-TLR7/8_ag [BL153]; | |
| **GM-CSF DC** | ^{Cy5}HSA [LH013]; | **15** |
| | ^{Cy5}HSA-TM₄₂[LH050]; | |
| | ^{Cy5}HSA-TM₄₂[BL149]; | |
| | ^{Cy5}HSA-TM₄₂[BL149] + ms serum | |
| | ^{Cy5}HSA-TM₄₂[BL149];+ inactivated ms serum | |
| **GM-CSF DC** | ^{Cy5}HSA-TM₄₂; ^{Cy5}HSA xxx | **16** |
| **DC (NPC)** | ^{Cy5}HSA-TM₁; ^{Cy5}HSA-TM₉; ^{Cy5}HSA-TM₂₀; ^{Cy5}HSA-TM₃₄; | **13** |
| | ^{Cy5}HSA-DBCO₄₀ | |
| **LSEC (NPC)** | ^{Cy5}HSA-TM₁; ^{Cy5}HSA-TM₉; ^{Cy5}HSA-TM₂₀; ^{Cy5}HSA-TM₃₄; | **13** |
| | ^{Cy5}HSA-DBCO₄₀ | |
| **Kupffer cells (NPC)** | ^{Cy5}HSA-TM₁; ^{Cy5}HSA-TM₉; ^{Cy5}HSA-TM₂₀; ^{Cy5}HSA-TM₃₄; | **13** |
| | ^{Cy5}HSA-DBCO₄₀ | |
| **T cell proliferation assay** | ^{Cy5}HSA [LH013]; | **17** |
| | ^{Cy5}HSA-TM₄₂[BL149]; | |
| | ^{Cy5}HSA-TM₄₂-TLR7/8_ag[BL153]; | |
| **FLT3L cDC1** | ^{Cy5}HSA [LH013]; | **18** |
| | ^{Cy5}HSA-TM₄₂[BL149]; | |
| | ^{Cy5}HSA-TM₄₂-TLR7/8_ag[BL153]; | |
| **FLT3L cDC2** | ^{Cy5}HSA [LH013]; | **19** |
| | ^{Cy5}HSA-TM₄₂[BL149]; | |
| | ^{Cy5}HSA-TM₄₂-TLR7/8_ag[BL153]; | |
| **FLT3L pDC** | ^{Cy5}HSA [LH013]; | **20** |
| | ^{Cy5}HSA-TM₄₂[BL149]; | |
| | ^{Cy5}HSA-TM₄₂-TLR7/8_ag[BL153]; | |
| **spleen DC** | ^{Cy5}HSA [LH013]; | **21** |
| | ^{Cy5}HSA-TM₄₂[BL149]; | |
| | ^{Cy5}HSA-TM₄₂-TLR7/8_ag[BL153]; | |
| **spleen T cells** | ^{Cy5}HSA [LH013]; | **22** |
| | ^{Cy5}HSA-TM₄₂[BL149]; | |
| | ^{Cy5}HSA-TM₄₂-TLR7/8_ag[BL153]; | |
| **spleen Mac** | ^{Cy5}HSA [LH013]; | **23** |
| | ^{Cy5}HSA-TM₄₂[BL149]; | |
| | ^{Cy5}HSA-TM₄₂-TLR7/8_ag[BL153]; | |
| **spleen PMN** | ^{Cy5}HSA [LH013]; | **24** |
| | ^{Cy5}HSA-TM₄₂[BL149]; | |
| | ^{Cy5}HSA-TM₄₂-TLR7/8_ag[BL153]; | |
| **spleen B cells** | ^{Cy5}HSA [LH013]; | **25** |
| | ^{Cy5}HSA-TM₄₂[BL149]; | |
| | ^{Cy5}HSA-TM₄₂-TLR7/8_ag[BL153]; | |
| **DC (NPC)** | ^{Cy5}HSA [LH013]; | **26** |
| | ^{Cy5}HSA-TM₄₂[BL149]; | |
| | ^{Cy5}HSA-TM₄₂-TLR7/8_ag[BL153]; | |
| **Kupffer cell (NPC)** | ^{Cy5}HSA [LH013]; | **27** |
| | ^{Cy5}HSA-TM₄₂[BL149]; | |
| | ^{Cy5}HSA-TM₄₂-TLR7/8_ag[BL153]; | |
| **LSEC (NPC)** | ^{Cy5}HSA [LH013]; | **28** |
| | ^{Cy5}HSA-TM₄₂[BL149]; | |
| | ^{Cy5}HSA-TM₄₂-TLR7/8_ag[BL153]; | |
| **M2-BMDM** | ^{Cy5}HSA [LH013]; | **29** |
| | ^{Cy5}HSA-TM₄₂[LH050]; | |
| | ^{Cy5}HSA-TM₄₂-CuB[BL151]; | |
| **GM-CSF & FLT3L DC** | ^{Cy5}HSA-TM₄₂[LH050]; | **30** |
| | ^{Cy5}HSA-TM₄₂-TLR7/8_ag[BL153]; | |
| | ^{Cy5}HSA-TM₄₂-TLR7/8_ag[BL153] + ms serum | |
| | ^{Cy5}HSA-TM₄₂-TLR7/8_ag[BL153];+ inactivated ms serum | |

### Methods (in vitro)

*Cell culture.* Murine bone marrow cells were seeded in 12-well suspension culture plates (Greiner Bio-One) in culture medium (IMDM, 2 mM L-glutamine, 100 U/mL penicillin, 100 µg/mL streptomycin (all from Sigma-Aldrich, Deisenhofen, Germany) and 50 µM β-mercaptoethanol (Roth) containing 5% FBS (PAN-Biotech, Aidenbach, Germany)) supplemented with recombinant murine GM-CSF (10 ng/mL; Miltenyi, Bergisch Gladbach, Germany) or FLT3-Ligand (250 ng/ml, Biolegend, San Diego, CA). Culture media was replenished on days 3 and 6 of culture of GM-CSF supplemented cell culture.

*In vitro differentiation*/*M2-polarization of bone marrow-derived macrophages.* Cryopreserved murine bone marrow-derived monocytes (BMDMs) were thawed and transferred to a 15 ml falcon tube containing DMEM, followed by centrifugation with 15000 rpm for 5 minutes. The supernatant was discarded and the cell pellet was resuspended in DMEM containing M-CSF (25 ng/µl). The cell suspensions were seeded into three non-collagenous petri dishes. The petri dishes were placed into the CO₂ Incubator. Differentiation of bone marrow-derived monocytes to matured macrophages was completed after approximately 1 week by regularly stimulating the cells with M-CSF (25 ng/ml) diluted in DMEM for 3 to 4 times throughout the week. The cells were counted with the Neubauer improved hemocytometer under the microscope. Afterwards, the cell suspension was diluted in fresh DMEM and divided by transferring the cell suspension to different falcon tubes. Macrophage maturation was followed by polarization towards M2 phenotype by stimulating with IL-4 (20 ng/µl) and IL-13 (20 ng/µl) cytokines. The cells were seeded into 12 well-plates with a cell number of 0.25 million cells per well. The 12-well plates were placed into the CO₂ incubator. After 24 hours, transfection with the drugs was carried out on the following day.

*Spleen cell and liver NPC isolation.* Murine spleens were mechanically disrupted with a pestle and pressed through a cell strainer with a pore size of 40 µM (Greiner Bio-One, Frickenhausen, Germany) to obtain a single-cell suspension. Spleen cells (2 × 10⁶/500 µL) were cultured in culture medium in FACS tubes.

For enrichment of liver NPC, mice were sacrificed by cervical dislocation and the liver was removed. For enzyme-based liver dissociation, a special enzyme mixture (Liver dissociation kit; Miltenyi Biotec, Bergisch-Gladbach, Germany) was prepared as recommended by the manufacturer. The enzyme mixture were preincubated for 15 min in C tubes (Miltenyi Biotec). Subsequently, livers were cut in pieces and transferred into prepared C tubes and placed into a gentleMACS Dissociator. The tissue was minced (m_liver_03). Following this, the cell suspension was incubated for 30 min at 37 °C under continuous shaking. Then, C tubes were again placed on the gentleMACS Dissociator and mixed (program m_liver_04). Samples were filtered using a Cell Strainer (100 µm; Sarstedt) and the liver NPCs were enriched by density centrifugation. Cells were resusepended using 30% Histodenz-HBSS and a HBSS layer was placed on top (both from Sigma-Aldrich, St. Louis, MO, USA). Then, gradient centrifugation (1500 × g, 20 min, 4 °C, without break and acceleration) was performed and liver NPC were retrieved from the interphase. Cells were cultured in HEPES buffered RPMI 1640 medium (10% FBS, 100 U/mL penicillin, 100 µg/mL streptomycin, 1 mM L-glutamine, 1% essential and non-essential amino acids, 50 µM β- mercaptoethanol) and placed in 96-well plates at a concentration of 4 × 10⁵/200 µl.

*Real-time quantitative PCR.* Total RNA was isolated using the NEB Monarch Mini Prep Kit (New England Biolabs, Ipswitch, USA) and 5 ng of total RNA was used for the One-Step qPCR Quantitative real-time PCR (Luna Universal One-Step qPCR-Kit), which was conducted using validated SYBR Green gene expression sets for mouse CD206, iNOS, YM1, TNFa and Arg-1 on a Step One Real-Time PCR System (Life Technologies, Carlsbad, CA, USA). Beta-Actin microglobulin (*Actinb*) served as an endogenous control for internal normalization. Data were analyzed using the ΔΔ-Ct method. The fold change was calculated as 2-ΔΔCt.

*Flow cytometry.* Samples were harvested, washed with staining buffer (PBS, 1% FBS, 0.5 mM EDTA) and incubated with rat anti-mouse CD16/CD32 antibody (clone 2.4G2; 15 min, 4°C) to prevent antibody binding to Fcγ receptors. Following this, samples were incubated with fluorescence-labeled antibodies (20 min, 4 °C). Then, samples were washed with PBS and incubated with FVD to identify live/dead cells. Measurements were carried out using an Attune NxT flow cytometer and data were analyzed using Attune NxT software (both Thermo Fisher, Waltham, MA, USA)

*T cell proliferation* assay. GM-CSF differentiated BMDCs were incubated with 5 µg/ml OVA protein. After 3 h nanocarriers or TLR7/8 agonist (1 µM) were added at different concentrations as indicated. The following day, samples were harvested, washed and resuspended in culture medium w/o GM-CSF. BMDCs were seeded in wells (2 × 10⁴/200 µL in triplicates) of 96-well plates (Greiner Bio-One) and were serially titrated five times (1:2). Splenic OVA peptide-specific CD4+ (OT-II) and CD8⁺ T cells (OT-I) were immunomagnetically sorted and enriched as recommended by the manufacturer (Miltenyi Biotec) and added to serially diluted BMDCs at a concentration of 5 × 10⁴ cells/100 µL. After 3-4 days of co-culture, ³H-thymidine (0.5 pCi/well) was introduced to the co-cultures for 16-18 h to assess T-cell proliferation. For measuring, cell lysates were transferred onto glass fiber filter mats (Harvester 96; TomTec, Hamden, CT, USA) and the genomically incorporated radioactivity was quantified using a microplate β-counter (1450 MicroBeta Trilux; Perkin Elmer, Waltham, MA, USA).

***Materials.*** PE- or PE-eFI610-labeled anti-CD11c (clone N418), PE-eFI610-MHCII (M5/114.15.2), PE-Cy7- or FITC-CD86 (GL-1), V500-CD3 (500A2), SB600-CD11b (M1/70), PerCP-Cy5.5-CD19 (1D3), PE-Cy7-NK1.1 (PK136), PE-eFI610-Ly6G (1A8-L6g), AF488-CD209a (MMD3), SB702-CD45 (30-F11), eFI450-F4/80 (BM8), PE-CD32b (AT130-2), BV421-XCR1 (ZET), HorizonV500-CD45R/B220 (RA3-6B2), Pe-Cy7-CD172a/Sirp1α (P84) and eFI780-FVD used for flow cytometric analysis were purchased from BD Biosciences (Franklin Lakes, NJ, USA), BioLegend (San Diego, CA, USA) or ThermoFisher (Waltham, MA, USA).

## Claims

1. A HSA-nanocarrier system for drug delivery into a target cell, wherein said nanocarrier system comprises:
A) a human serum albumin (HSA);
B) a plurality of mannose-based targeting structures covalently bound to said HSA;
C) at least one immunomodulatory drug covalently bound to said HSA;
wherein said HSA nanocarrier system has a diameter in the range comprised between 4 nm and 150 nm, as measured by dynamic light scattering.

2. The HSA-nanocarrier system according to claim 1, wherein the mannose-based targeting structure of the plurality of mannose-based targeting structures is selected from the group consisting of ManpNs, ManpsNs, (Man*p*)*₃*N₃, and (Man*p₃*)₃N₃, preferably Man*p₃*N₃.

3. The HSA-nanocarrier system according to claim 1 or 2, wherein the plurality of mannose-based targeting structures is covalently bound to the HSA via an interconnecting molecule and the molar ratio of interconnecting molecule:HSA is comprised between 3 and 59, and/or
wherein the molar mannose-based targeting structure:HSA ratio is comprised between 1 and 59.

4. The HSA-nanocarrier system according to any one of claims 1 - 3, wherein the at least one immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

5. The HSA nanocarrier system according to any one of claims 1 - 4, wherein the at least one immunomodulatory drug is covalently bound to said HSA by a traceless, self-immolative drug linker comprising a thiol-reactive functional group, wherein the traceless, self-immolative linker is selected pyridinyldithiol-ethoxy-carbonyl or mc-vc-pabc linker (maleimidocaproyl (mc) - valine-citrulline (vc) -para-amino benzyloxycarbonyl (PABC)).

6. The HSA nanocarrier system according to any one of claims 1 - 5, wherein the HSA-nanocarrier system further comprises:
D) a fluorescent dye covalently bound to said HSA, wherein the fluorescent dye comprises a maleimide as thiol-reactive functional group or an NHS-ester as amine-reactive functional group, and wherein the fluorescent dye emits light in a range of 400 nm to 850 nm.

7. The HSA nanocarrier system according to claim 6, wherein the fluorescent dye is a fluorescent dye maleimide selected from the group consisting of sulfo-cyanin-maleimide, Alexa Fluor-maleimide, and cyanin-maleimide.

8. The HSA nanocarrier system according to any one of claims 1 - 7, wherein said HSA nanocarrier system comprises:
- trimannose bound via an NHS-PEGn-DBCO interconnecting molecule to HSA at a molar trimannose:HSA range comprised between 1 and 59;
- Cucurbitacin B or TLR7/8 agonist as an immunomodulatory drug at a molar drug:HSA range preferably comprised between 5 and 27, wherein the immunomodulatory drug is covalently bound to said HSA by a traceless, self-immolative pyridinyldithio-ethoxy-carbonyl drug linker;
- optionally a sulfo-Cyanin5-maleimide dye or a sulfo-Cyanin7.5-maleimide dye conjugated to said HSA.

9. A pharmaceutical composition comprising the HSA nanocarrier system according to any one of claims 1 - 8 and a pharmaceutical acceptable excipient.

10. A method to manufacture a HSA nanocarrier system according to any one of claims 1 - 9, wherein the method comprises the following steps:
i.) providing monomolecular native HSA, wherein the monomolecular native HSA is optionally labeled with a fluorescent dye;
ii.) attaching an interconnecting molecule to the HSA;
iii.) covalently binding of a mannose-based targeting structure to the interconnecting molecule to obtain mannosylated HSA;
iv.) denaturing of the mannosylated HSA obtained at step iii.;
v.-a) covalently binding of at least one immunomodulatory drugs to said denatured mannosylated HSA, wherein the one or more immunomodulatory drugs are attached to a drug linker comprising a thiol-reactive functional group;
v.-b) capping of unreacted thiols, preferably with N-ethylmaleimide;
v.-c) washing the denatured nanocarrier system obtained in step v.-b;
vi.) backfolding of the denatured nanocarrier system via rapid dilution procedure;
vii.) obtaining a monomolecular HSA nanocarrier system.

11. The method to manufacture a HSA nanocarrier system according to claim 10, wherein the mannose-based targeting structure is selected from the group consisting of ManpNs, ManpsNs, (Man*p*)*₃*N₃, and (Man*p₃*)₃N₃.

12. The method to manufacture a HSA nanocarrier system according to claim 10 or 11, wherein the at least one immunomodulatory drug is selected from the group consisting of TLR agonist, TLR7/8 agonist, PARP inhibitor, JAK inhibitor, STAT3 inhibitor, Cucurbitacin B, ERAP2 inhibitor, IDO1 inhibitor, curcuminoids, gingerol, and silibinin.

13. The method to manufacture a HSA nanocarrier system according to any one of claims 10 - 12, wherein the at least one immunomodulatory drug is covalently bound to said HSA by a drug linker selected from the group consisting of pyridinyldithio-ethoxy-carbonyl-group, maleimide, mc-vc-pabc linker, thiosulfonate linker, hydrazide-maleimide linker, iodoacetamide linker, methanethiosulfonate linker.

14. The method to manufacture a HSA nanocarrier system according to any one of claims 10 - 13, wherein the HSA is labeled with a fluorescent dye emitting fluorescence in a range comprised between 400 and 850 nm.

15. The method to manufacture a HSA nanocarrier system according to claim any one of claims 10 - 14, wherein the HSA is labeled with a fluorescent dye via a maleimide and the method further comprises the following step i.1) before step ii.):
i.1) subjecting the HSA labeled with a fluorescent dye maleimide to a ring-opening hydrolysis by adding a buffer at a pH above 9.
